# EUROPEAN PATENT SPECIFICATION

(11) **EP 2 489 738 B1**
(45) Date of publication and mention of the grant of the patent: **17.05.2017**
(21) Application number: 10823470.9
(22) Date of filing: 15.10.2010
(51) Int. Cl.: C12N 15/29, A01H 1/00, A01H 5/00, C12N 5/10

(54) **GENE CONTROLLING FLOWERING HABIT/CLEISTOGAMY IN PLANTS, AND USE THEREOF**
GEN ZUR STEUERUNG DES BLÜHVERHALTENS/DER KLEISTOGAMIE VON PFLANZEN UND VERWENDUNG DAVON
GÈNE RÉGULANT LE COMPORTEMENT DE FLORAISON/LA CLÉISTOGAMIE DE PLANTES, ET SON UTILISATION

(30) Priority: 16.10.2009 JP 2009239162
(43) Date of publication of application: 22.08.2012
(73) Proprietor: National Institute Of Agrobiological Sciences, Ibaraki 305-8602 (JP)
(72) Inventor: KOMATSUDA Takao, Tsukuba-shi Ibaraki 305-8602 (JP); MATSUMOTO Takashi, Tsukuba-shi Ibaraki 305-8602 (JP)
(74) Representative: Cohausz & Florack
(86) International application number: PCT/JP2010/068164
(87) International publication number: WO 2011/046202

(56) References cited:
- JP-A- 2004 180 570
- JP-A- 2005 229 854
- SHUNZONG NING ET AL: "Structure, transcription and post-transcriptional regulation of the bread wheat orthologs of the barley cleistogamy gene Cly1", TAG THEORETICAL AND APPLIED GENETICS, vol. 126, no. 5, 1 May 2013 (2013-05-01), pages 1273-1283, XP055076655, ISSN: 0040-5752, DOI: 10.1007/s00122-013-2052-6
- CHEN X: 'A microRNA as a translational repressor of APETALA2 in Arabidopsis flower development.' SCIENCE vol. 303, 2004, pages 2022 - 2025, XP008156015
- MLOTSHWA S ET AL.: 'Floral patterning defects induced by Arabidopsis APETALA2 and microRNA172 expression in Nicotiana benthamiana.' PLANT MOL. BIOL. vol. 61, 2006, pages 781 - 793, XP019405478
- INTERNATIONAL RICE GENOME SEQUENCING PROGECT: 'The map-based sequence of the rice genome' NATURE vol. 436, 2005, pages 793 - 800, XP002442031
- TANAKA T ET AL.: 'The Rice Annotation Project Database (RAP-DB): 2008 update' NUCL. ACIDS RES. vol. 36, 2008, pages D1028 - D1033, XP008156018
- TAKAO KOMATSUDA: 'Omugi no Ho no Keitai Keisei ni Kan'yo suru Idenshi no Tanri to Kozo Kaiseki' KENKYU SEIKA DAI 474 SHU 'GENOME IKUSHU NI YORU KORITSUTEKI HINSHU IKUSEI GIJUTSU NO KAIHATSU -TAYOSEI GENOME KAISEKI KENKYU-' 20 February 2009, pages 69 - 73, XP008160457
- NAIR S K ET AL.: 'Cleistogamous flowering in barley arises from the suppression of microRNA- guided HvAP2 mRNA cleavage.' PROC. NATL. ACAD. SCI. USA vol. 107, 05 January 2010, pages 490 - 495, XP008156020
- TURUSPEKOV Y ET AL.: 'An inverted and micro- colinear genomic regions of rice and barley carrying the clyl gene for cleistogamy.' BREED. SCI. vol. 59, no. 5, 10 December 2009, pages 657 - 663, XP008156027

### SEQUENCE LISTING

<110> National Institute of Agrobiological Sciences
   <120> A gene for controlling cleistogamy/chasmogamy and usage thereof
   <130> dokugai127
   <150> JP 09/239162
   <151> 2009-10-16
   <160> 16
   <170> PatentIn version 3.1
<210> 1
   <211> 1464
   <212> DNA
   <213> Hordeum vulgare
   <220>
   <221> CDS
   <222> (1) .. (1461)
<400> 1
   atg tgg gat ctc aac gac tcg ccg gcg gcc gag ggg ccg ccg ctg tcc 48
   Met Trp Asp Leu Asn Asp Ser Pro Ala Ala Glu Gly Pro Pro Leu Ser
   1 5 10 15
   ccg tcc gtg gac gac tcc ggc gcc tcc tcc tcg tct gcc gcc gcg gtg 96
   Pro Ser Val Asp Asp Ser Gly Ala Ser Ser Ser Ser Ala Ala Ala Val
   20 25 30
   gtc gag ata ccg gac gac gcc gag gac gac tcc gcc gag gcc gtc gtc 144
   Val Glu Ile Pro Asp Asp Ala Glu Asp Asp Ser Ala Glu Ala Val Val
   35 40 45
   atg cgc cag ttc ttc ccc ccg gcc gcc ccg ggc gag ggc ggc ccc ggc 192
   Met Arg Gln Phe Phe Pro Pro Ala Ala Pro Gly Glu Gly Gly Pro Gly
   50 55 60
   aac ggg aac gac cgc gcc gcg tgg ctc cgc ctg gcc ggc gcc ccc gcg 240
   Asn Gly Asn Asp Arg Ala Ala Trp Leu Arg Leu Ala Gly Ala Pro Ala
   65 70 75 80
   ccc acc gtg gcc gcg gcc gcc gga gga gga aca gga ggc ccc gcg gcg 288
   Pro Thr Val Ala Ala Ala Ala Gly Gly Gly Thr Gly Gly Pro Ala Ala
   85 90 95
   gcg tcg gcg gcg gcc aag aag agc cgg cgc ggt ccc cgt tcc cgc agc 336
   Ala Ser Ala Ala Ala Lys Lys Ser Arg Arg Gly Pro Arg Ser Arg Ser
   100 105 110
   tcg cag tac cgc ggc gtc acc ttc tac cgc cgg acg ggc cgg tgg gag 384
   Ser Gln Tyr Arg Gly Val Thr Phe Tyr Arg Arg Thr Gly Arg Trp Glu
   115 120 125
   tcg cac ata tgg gat tgc ggc aag cag gtc tat ctg ggt gga ttc gac 432
   Ser His Ile Trp Asp Cys Gly Lys Gln Val Tyr Leu Gly Gly Phe Asp
   130 135 140
   act gct cat gcg gcg gct cgg gcg tac gat cgg gcg gcg atc aag ttc 480
   Thr Ala His Ala Ala Ala Arg Ala Tyr Asp Arg Ala Ala Ile Lys Phe
   145 150 155 160
   cgc ggc atg gag gcc gac atc aat ttc agc ctg gag gac tac gac gac 528
   Arg Gly Met Glu Ala Asp Ile Asn Phe Ser Leu Glu Asp Tyr Asp Asp
   165 170 175
   atc aag cag atg ggc aac ctg acc aag gag gag ttc gtc cac gtg ctc 576
   Ile Lys Gln Met Gly Asn Leu Thr Lys Glu Glu Phe Val His Val Leu
   180 185 190
   cgg cgg cag agc acg ggg ttc ccc cgg ggg agc tcc aag tac agg ggc 624
   Arg Arg Gln Ser Thr Gly Phe Pro Arg Gly Ser Ser Lys Tyr Arg Gly
           195                 200                 205
   gtc acg ctc cac aag tgc ggc agg tgg gag gcg cgg atg ggc cag ttc      672
   Val Thr Leu His Lys Cys Gly Arg Trp Glu Ala Arg Met Gly Gln Phe
       210                 215                 220
   ctc ggc aag aag tac gtc tac ttg ggg ctg ttc gat acc gag gag gaa      720
   Leu Gly Lys Lys Tyr Val Tyr Leu Gly Leu Phe Asp Thr Glu Glu Glu
   225                 230                 235                 240
   gct gcc agg tcg tac gac cgc gct gcc atc aag tgc aac ggc aag gat      768
   Ala Ala Arg Ser Tyr Asp Arg Ala Ala Ile Lys Cys Asn Gly Lys Asp
                   245                 250                 255
   gcg gtc acc aac ttc gat ccc agc acc tac gcc gag gag ttc gag ccg      816
   Ala Val Thr Asn Phe Asp Pro Ser Thr Tyr Ala Glu Glu Phe Glu Pro
               260                 265                 270
   gcg gct tcg acc ggc gac gcg gag cag cag aac ctg gac ctg tcg ctg      864
   Ala Ala Ser Thr Gly Asp Ala Glu Gln Gln Asn Leu Asp Leu Ser Leu
           275                 280                 285
   ggg agc tcg gcg ggg tcg aac aag agg ggc agc ctc gac ggc ggc ggc      912
   Gly Ser Ser Ala Gly Ser Asn Lys Arg Gly Ser Leu Asp Gly Gly Gly
       290                 295                 300
   atg gac gac gac ggc gcg gcg ggg tcc gac cag cgc gtc ccc atg gcc      960
   Met Asp Asp Asp Gly Ala Ala Gly Ser Asp Gln Arg Val Pro Met Ala
   305                 310                 315                 320
   ttc gag ctc gac tgg cac acg gcg gcg gcg cgc agc acc aag gcc aag     1008
   Phe Glu Leu Asp Trp His Thr Ala Ala Ala Arg Ser Thr Lys Ala Lys
                   325                 330                 335
   ttc gac cag aac tcg gcg cgt cat cag atg ccc cct cca gcc ctg caa     1056
   Phe Asp Gln Asn Ser Ala Arg His Gln Met Pro Pro Pro Ala Leu Gln
               340                 345                 350
   ctg caa gcc ccc cac atg cag ttc agt ccc agg cat cac caa ttc gtg     1104
   Leu Gln Ala Pro His Met Gln Phe Ser Pro Arg His His Gln Phe Val
           355                 360                 365
   ggc aac gcc gat ccg ggg aca gcg gga ggc ctg tcg ctg acg gtc ggc     1152
   Gly Asn Ala Asp Pro Gly Thr Ala Gly Gly Leu Ser Leu Thr Val Gly
       370                 375                 380
   gcc ggc gcc ggg ggc ggg caa tgg cct ccg cct ccg cct ccc cac cac     1200
   Ala Gly Ala Gly Gly Gly Gln Trp Pro Pro Pro Pro Pro Pro His His
   385                 390                 395                 400
   tac cag ccg ccg cat ccg cag cag cac cac cag cag cag caa cag agg     1248
   Tyr Gln Pro Pro His Pro Gln Gln His His Gln Gln Gln Gln Gln Arg
                   405                 410                 415
   ctg cag cac ggc tgg ggc aac gtc gtc ccc ggc acg agc tgg cag ccg     1296
   Leu Gln His Gly Trp Gly Asn Val Val Pro Gly Thr Ser Trp Gln Pro
               420                 425                 430
   gtc cag ccg ccg ccg ccg ccg cac cac cag gcg ggg ccg gcg ccg aac     1344
   Val Gln Pro Pro Pro Pro Pro His His Gln Ala Gly Pro Ala Pro Asn
           435                 440                 445
   aac gct gcc gcc gcc gca gca gca gca gca gcg tca tca cga ttc cca     1392
   Asn Ala Ala Ala Ala Ala Ala Ala Ala Ala Ala Ser Ser Arg Phe Pro
       450                 455                 460
   ccc tac atc gcc acg cag gcg cag agc tgg ctc cag aag aac ggg ttc     1440
   Pro Tyr Ile Ala Thr Gln Ala Gln Ser Trp Leu Gln Lys Asn Gly Phe
   465                 470                 475                 480
   cac tcc ctg gcc aga ccc acc tag                                     1464
   His Ser Leu Ala Arg Pro Thr
                   485
<210> 2
   <211> 2691
   <212> DNA
   <213> Hordeum vulgare
<220>
   <221> exon
   <222> (1) .. (395)
<220>
   <221> Intron
   <222> (396) .. (668)
<220>
   <221> exon
   <222> (669) .. (694)
<220>
   <221> Intron
   <222> (695) .. (790)
<220>
   <221> exon
   <222> (791) .. (821)
<220>
   <221> Intron
   <222> (822) .. (916)
<220>
   <221> exon
   <222> (917) .. (1001)
<220>
   <221> Intron
   <222> (1002) .. (1083)
<220>
   <221> exon
   <222> (1084) .. (1229)
<220>
   <221> Intron
   <222> (1230) .. (1423)
<220>
   <221> exon
   <222> (1424) .. (1468)
<220>
   <221> Intron
   <222> (1469) .. (1600)
<220>
   <221> exon
   <222> (1601) .. (1691)
<220>
   <221> Intron
   <222> (1692) .. (1836)
<220>
   <221> exon
   <222> (1837) .. (2025)
<220>
   <221> Intron
   <222> (2026) .. (2132)
<220>
   <221> exon
   <222> (2133) .. (2222)
<220>
   <221> Intron
   <222> (2223) .. (2325)
<220>
   <221> exon
   <222> (2326) .. (2691)
<400> 2
   atg tgg gat ctc aac gac tcg ccg gcg gcc gag ggg ccg ccg ctg tcc 48
   Met Trp Asp Leu Asn Asp Ser Pro Ala Ala Glu Gly Pro Pro Leu Ser
   1 5 10 15
   ccg tcc gtg gac gac tcc ggc gcc tcc tcc tcg tct gcc gcc gcg gtg 96
   Pro Ser Val Asp Asp Ser Gly Ala Ser Ser Ser Ser Ala Ala Ala Val
   20 25 30
   gtc gag ata ccg gac gac gcc gag gac gac tcc gcc gag gcc gtc gtc 144
   Val Glu Ile Pro Asp Asp Ala Glu Asp Asp Ser Ala Glu Ala Val Val
   35 40 45
   atg cgc cag ttc ttc ccc ccg gcc gcc ccg ggc gag ggc ggc ccc ggc 192
   Met Arg Gln Phe Phe Pro Pro Ala Ala Pro Gly Glu Gly Gly Pro Gly
   50 55 60
   aac ggg aac gac cgc gcc gcg tgg ctc cgc ctg gcc ggc gcc ccc gcg 240
   Asn Gly Asn Asp Arg Ala Ala Trp Leu Arg Leu Ala Gly Ala Pro Ala
   65 70 75 80
   ccc acc gtg gcc gcg gcc gcc gga gga gga aca gga ggc ccc gcg gcg 288
   Pro Thr Val Ala Ala Ala Ala Gly Gly Gly Thr Gly Gly Pro Ala Ala
   85 90 95
   gcg tcg gcg gcg gcc aag aag agc cgg cgc ggt ccc cgt tcc cgc agc 336
   Ala Ser Ala Ala Ala Lys Lys Ser Arg Arg Gly Pro Arg Ser Arg Ser
   100 105 110
   tcg cag tac cgc ggc gtc acc ttc tac cgc cgg acg ggc cgg tgg gag 384
   Ser Gln Tyr Arg Gly Val Thr Phe Tyr Arg Arg Thr Gly Arg Trp Glu
   115 120 125
   tcg cac ata tg gtaagctccc gccgccgctc cgctcctttc ctttcctttc 435
   Ser His Ile Trp
   130
   tcgaaagctt aaggaaggaa ggagagagag tgcagcaaaa gaagagaaga gaagagaaga 495
   tggatggatg gatgggtttg gtattggatt cgtcttgttt tgggcattcc atgcctctgc 555
   tccttttacc tttcatagca acatcttatc aaacttattc cccttccttc cttgtttctc 615
   tctcttcccc cctctcctct cctcttctca ccccattctt ttgtgcaatg cag g gat 672
   Asp
   tgc ggc aag cag gtc tat ctg g gtaagtttcc tccatccccg tcttgtccac 724
   Cys Gly Lys Gln Val Tyr Leu
   135 140
   acatccatac ttggggtgcg aaatcgatcg ccaattggag ctcacaatgt cactgacggc 784
   ctgcag gt gga ttc gac act gct cat gcg gcg gct cg gtacgataaa 831
   Gly Gly Phe Asp Thr Ala His Ala Ala Ala Arg
   145 150
   gatctcatac caaccgattt gacagtacca atcaagctct cttctttact gatttcttct 891
   tcttcatcat catctgcggc cgcag g gcg tac gat cgg gcg gcg atc aag ttc 944
   Ala Tyr Asp Arg Ala Ala Ile Lys Phe
   155 160
   cgc ggc atg gag gcc gac atc aat ttc agc ctg gag gac tac gac gac 992
   Arg Gly Met Glu Ala Asp Ile Asn Phe Ser Leu Glu Asp Tyr Asp Asp
   165 170 175
   atc aag cag gtgagcgacg cccgatcgat caacacgagc tagcgcttgt 1041
   Ile Lys Gln
   tgctcgcaga atcgtaacgt gtttgccctg gtttgcttgc ag atg ggc aac ctg      1095
                                                  Met Gly Asn Leu
                                                  180
   acc aag gag gag ttc gtc cac gtg ctc cgg cgg cag agc acg ggg ttc     1143
   Thr Lys Glu Glu Phe Val His Val Leu Arg Arg Gln Ser Thr Gly Phe
       185                 190                 195
   ccc cgg ggg agc tcc aag tac agg ggc gtc acg ctc cac aag tgc ggc     1191
   Pro Arg Gly Ser Ser Lys Tyr Arg Gly Val Thr Leu His Lys Cys Gly
   200                 205                 210                 215
   agg tgg gag gcg cgg atg ggc cag ttc ctc ggc aag aa  gtatgtgctc      1239
   Arg Trp Glu Ala Arg Met Gly Gln Phe Leu Gly Lys Lys
                   220                 225
   ctccaccaac caccatcgcc accctccttc ctcccttctc ctccaatcga tggctcgaat   1299
   ttttgctctg ctcgtctgcc tgctcctgtc gttgcgctca aagttgcagc atctccctgc   1359
   taactagcct gacggcatgg catggcatgg cagcggcact aatgagaatc tttgtgcaac   1419
   gcag g tac gtc tac ttg ggg ctg ttc gat acc gag gag gaa gct gcc      1466
          Tyr Val Tyr Leu Gly Leu Phe Asp Thr Glu Glu Glu Ala Ala
              230                 235                 240
   ag  gtagtaaaaa ctgaaaaatt attgggcatg cactgtgcgc gcgtcctcca          1518
   Arg
   tccattgcta gctttagctt tgctcgggct ggggttgggc ggtgaactga ttgatgtctc   1578
   gtgtgtctct gtctctgggc ag g tcg tac gac cgc gct gcc atc aag tgc      1628
                              Ser Tyr Asp Arg Ala Ala Ile Lys Cys
                                  245                 250
   aac ggc aag gat gcg gtc acc aac ttc gat ccc agc acc tac gcc gag     1676
   Asn Gly Lys Asp Ala Val Thr Asn Phe Asp Pro Ser Thr Tyr Ala Glu
           255                 260                 265
   gag ttc gag ccg gcg ggtcagtaat aatcttgtta tattcatcgc gcactgttca     1731
   Glu Phe Glu Pro Ala
       270
   tctgtgattt tgatttggcc accataggat tatgcggcat acgtatgttt cttccggttc   1791
   gttcactgac cttgatcttg atcttgggtg ggcaatgctc tgcca gct tcg acc ggc   1848
                                                     Ala Ser Thr Gly
                                                             275
   gac gcg gag cag cag aac ctg gac ctg tcg ctg ggg agc tcg gcg ggg     1896
   Asp Ala Glu Gln Gln Asn Leu Asp Leu Ser Leu Gly Ser Ser Ala Gly
           280                 285                 290
   tcg aac aag agg ggc agc ctc gac ggc ggc ggc atg gac gac gac ggc     1944
   Ser Asn Lys Arg Gly Ser Leu Asp Gly Gly Gly Met Asp Asp Asp Gly
       295                 300                 305
   gcg gcg ggg tcc gac cag cgc gtc ccc atg gcc ttc gag ctc gac tgg     1992
   Ala Ala Gly Ser Asp Gln Arg Val Pro Met Ala Phe Glu Leu Asp Trp
   310                 315                 320                 325
   cac acg gcg gcg gcg cgc agc acc aag gcc aag gtacacgccc caacttaacc   2045
   His Thr Ala Ala Ala Arg Ser Thr Lys Ala Lys
                   330                 335
   ttgaccaccc tgcaacaaac tactgctcct gcatcatagt ataatcaagc gttggaaaag   2105
   ctgagaatgg cgtaatttac tatgcag ttc gac cag aac tcg gcg cgt cat cag   2159
                                 Phe Asp Gln Asn Ser Ala Arg His Gln
                                         340                 345
   atg ccc cct cca gcc ctg caa ctg caa gcc ccc cac atg cag ttc agt     2207
   Met Pro Pro Pro Ala Leu Gln Leu Gln Ala Pro His Met Gln Phe Ser
                   350                 355                 360
   ccc agg cat cac caa gtgggtactt ttgccgctcc aagttggagc tcacgaattt     2262
   Pro Arg His His Gln
           365
   ttctatctct tctcttcttg gtaggcgcga attaacggtg gttgtttggt tcaaactgtg     2322
   cag ttc gtg ggc aac gcc gat ccg ggg aca gcg gga ggc ctg tcg ctg       2370
       Phe Val Gly Asn Ala Asp Pro Gly Thr Ala Gly Gly Leu Ser Leu
                   370                 375                 380
   acg gtc ggc gcc ggc gcc ggg ggc ggg caa tgg cct ccg cct ccg cct       2418
   Thr Val Gly Ala Gly Ala Gly Gly Gly Gln Trp Pro Pro Pro Pro Pro
               385                 390                 395
   ccc cac cac tac cag ccg ccg cat ccg cag cag cac cac cag cag cag       2466
   Pro His His Tyr Gln Pro Pro His Pro Gln Gln His His Gln Gln Gln
           400                 405                 410
   caa cag agg ctg cag cac ggc tgg ggc aac gtc gtc ccc ggc acg agc       2514
   Gln Gln Arg Leu Gln His Gly Trp Gly Asn Val Val Pro Gly Thr Ser
       415                 420                 425
   tgg cag ccg gtc cag ccg ccg ccg ccg ccg cac cac cag gcg ggg ccg       2562
   Trp Gln Pro Val Gln Pro Pro Pro Pro Pro His His Gln Ala Gly Pro
   430                 435                 440                 445
   gcg ccg aac aac gct gcc gcc gcc gca gca gca gca gca gcg tca tca       2610
   Ala Pro Asn Asn Ala Ala Ala Ala Ala Ala Ala Ala Ala Ala Ser Ser
                   450                 455                 460
   cga ttc cca ccc tac atc gcc acg cag gcg cag agc tgg ctc cag aag       2658
   Arg Phe Pro Pro Tyr Ile Ala Thr Gln Ala Gln Ser Trp Leu Gln Lys
               465                 470                 475
   aac ggg ttc cac tcc ctg gcc aga ccc acc tag                           2691
   Asn Gly Phe His Ser Leu Ala Arg Pro Thr
           480                 485
<210> 3
   <211> 487
   <212> PRT
   <213> Hordeum vulgare
<400> 3
   Met Trp Asp Leu Asn Asp Ser Pro Ala Ala Glu Gly Pro Pro Leu Ser
   1 5 10 15
   Pro Ser Val Asp Asp Ser Gly Ala Ser Ser Ser Ser Ala Ala Ala Val
   20 25 30
   Val Glu Ile Pro Asp Asp Ala Glu Asp Asp Ser Ala Glu Ala Val Val
   35 40 45
   Met Arg Gln Phe Phe Pro Pro Ala Ala Pro Gly Glu Gly Gly Pro Gly
   50 55 60
   Asn Gly Asn Asp Arg Ala Ala Trp Leu Arg Leu Ala Gly Ala Pro Ala
   65 70 75 80
   Pro Thr Val Ala Ala Ala Ala Gly Gly Gly Thr Gly Gly Pro Ala Ala
   85 90 95
   Ala Ser Ala Ala Ala Lys Lys Ser Arg Arg Gly Pro Arg Ser Arg Ser
   100 105 110
   Ser Gln Tyr Arg Gly Val Thr Phe Tyr Arg Arg Thr Gly Arg Trp Glu
   115 120 125
   Ser His Ile Trp Asp Cys Gly Lys Gln Val Tyr Leu Gly Gly Phe Asp
   130 135 140
   Thr Ala His Ala Ala Ala Arg Ala Tyr Asp Arg Ala Ala Ile Lys Phe
   145 150 155 160
   Arg Gly Met Glu Ala Asp Ile Asn Phe Ser Leu Glu Asp Tyr Asp Asp
   165 170 175
   Ile Lys Gln Met Gly Asn Leu Thr Lys Glu Glu Phe Val His Val Leu
   180 185 190
   Arg Arg Gln Ser Thr Gly Phe Pro Arg Gly Ser Ser Lys Tyr Arg Gly
           195                 200                 205
   Val Thr Leu His Lys Cys Gly Arg Trp Glu Ala Arg Met Gly Gln Phe
       210                 215                 220
   Leu Gly Lys Lys Tyr Val Tyr Leu Gly Leu Phe Asp Thr Glu Glu Glu
   225                 230                 235                 240
   Ala Ala Arg Ser Tyr Asp Arg Ala Ala Ile Lys Cys Asn Gly Lys Asp
                   245                 250                 255
   Ala Val Thr Asn Phe Asp Pro Ser Thr Tyr Ala Glu Glu Phe Glu Pro
               260                 265                 270
   Ala Ala Ser Thr Gly Asp Ala Glu Gln Gln Asn Leu Asp Leu Ser Leu
           275                 280                 285
   Gly Ser Ser Ala Gly Ser Asn Lys Arg Gly Ser Leu Asp Gly Gly Gly
       290                 295                 300
   Met Asp Asp Asp Gly Ala Ala Gly Ser Asp Gln Arg Val Pro Met Ala
   305                 310                 315                 320
   Phe Glu Leu Asp Trp His Thr Ala Ala Ala Arg Ser Thr Lys Ala Lys
                   325                 330                 335
   Phe Asp Gln Asn Ser Ala Arg His Gln Met Pro Pro Pro Ala Leu Gln
               340                 345                 350
   Leu Gln Ala Pro His Met Gln Phe Ser Pro Arg His His Gln Phe Val
           355                 360                 365
   Gly Asn Ala Asp Pro Gly Thr Ala Gly Gly Leu Ser Leu Thr Val Gly
       370                 375                 380
   Ala Gly Ala Gly Gly Gly Gln Trp Pro Pro Pro Pro Pro Pro His His
   385                 390                 395                 400
   Tyr Gln Pro Pro His Pro Gln Gln His His Gln Gln Gln Gln Gln Arg
                   405                 410                 415
   Leu Gln His Gly Trp Gly Asn Val Val Pro Gly Thr Ser Trp Gln Pro
               420                 425                 430
   Val Gln Pro Pro Pro Pro Pro His His Gln Ala Gly Pro Ala Pro Asn
           435                 440                 445
   Asn Ala Ala Ala Ala Ala Ala Ala Ala Ala Ala Ser Ser Arg Phe Pro
       450                 455                 460
   Pro Tyr Ile Ala Thr Gln Ala Gln Ser Trp Leu Gln Lys Asn Gly Phe
   465                 470                 475                 480
   His Ser Leu Ala Arg Pro Thr
                   485
<210> 4
   <211> 1464
   <212> DNA
   <213> Hordeum vulgare
<220>
   <221> CDS
   <222> (1) .. (1461)
   <223>
<400> 4
   atg tgg gat ctc aac gac tcg ccg gcg gcc gag ggg ccg ccg ctg tcc          48
   Met Trp Asp Leu Asn Asp Ser Pro Ala Ala Glu Gly Pro Pro Leu Ser
   1               5                   10                  15
   ccg tcc gtg gac gac tcc ggc gcc tcc tcc tcg tct gcc gcc gcg gtg          96
   Pro Ser Val Asp Asp Ser Gly Ala Ser Ser Ser Ser Ala Ala Ala Val
               20                  25                  30
   gtc gag ata ccc gac gac gcc gag gac gac tcc gcc gag gcc gtc gtc         144
   Val Glu Ile Pro Asp Asp Ala Glu Asp Asp Ser Ala Glu Ala Val Val
               35                      40                      45
   acg cgc cag ttc ttc ccc ccg gcc gcc ccg ggc gag ggc ggc ccc ggc      192
   Thr Arg Gln Phe Phe Pro Pro Ala Ala Pro Gly Glu Gly Gly Pro Gly
           50                      55                      60
   aac ggg aac gac cgc gcc gcg tgg ctc cgc ctg gcc ggc gcc ccc gcg      240
   Asn Gly Asn Asp Arg Ala Ala Trp Leu Arg Leu Ala Gly Ala Pro Ala
   65                  70                      75                  80
   ccc acc gtg gcc gcg gcc gcc gga gga gga aca gga ggc ccc gcg gcg      288
   Pro Thr Val Ala Ala Ala Ala Gly Gly Gly Thr Gly Gly Pro Ala Ala
                   85                  90                      95
   gcg tcg gcg gcg gcc aag aag agc cgg cgc ggt ccc cgt tcc cgc agc      336
   Ala Ser Ala Ala Ala Lys Lys Ser Arg Arg Gly Pro Arg Ser Arg Ser
               100                     105                     110
   tcg cag tac cgc ggc gtc acc ttc tac cgc cgg acg ggc cgg tgg gag      384
   Ser Gln Tyr Arg Gly Val Thr Phe Tyr Arg Arg Thr Gly Arg Trp Glu
           115                     120                     125
   tcg cac ata tgg gat tgc ggc aag cag gtc tat ctg ggt gga ttc gac      432
   Ser His Ile Trp Asp Cys Gly Lys Gln Val Tyr Leu Gly Gly Phe Asp
       130                     135                     140
   act gct cat gcg gcg gct cgg gcg tac gat cgg gcg gcg atc aag ttc      480
   Thr Ala His Ala Ala Ala Arg Ala Tyr Asp Arg Ala Ala Ile Lys Phe
   145                 150                     155                 160
   cgc ggc atg gag gcc gac atc aat ttc agc ctg gag gac tac gac gac      528
   Arg Gly Met Glu Ala Asp Ile Asn Phe Ser Leu Glu Asp Tyr Asp Asp
                   165                     170                 175
   atc aag cag atg ggc aac cta acc aag gag gag ttc gtc cac gtg ctc      576
   Ile Lys Gln Met Gly Asn Leu Thr Lys Glu Glu Phe Val His Val Leu
               180                     185                     190
   cgg cgg cag agc acg ggg ttc ccc cgg ggg agc tcc aag tac agg ggc      624
   Arg Arg Gln Ser Thr Gly Phe Pro Arg Gly Ser Ser Lys Tyr Arg Gly
           195                     200                     205
   gtc acg ctc cac aag tgc ggc agg tgg gag gcg cgg atg ggc cag ttc      672
   Val Thr Leu His Lys Cys Gly Arg Trp Glu Ala Arg Met Gly Gln Phe
       210                     215                     220
   ctc ggc aag aag tac gtc tac ttg ggg ctg ttc gat acc gag gag gaa      720
   Leu Gly Lys Lys Tyr Val Tyr Leu Gly Leu Phe Asp Thr Glu Glu Glu
   225                 230                     235                 240
   gct gcc agg tcg tac gac cgc gct gcc atc aag tgc aac ggc aag gat      768
   Ala Ala Arg Ser Tyr Asp Arg Ala Ala Ile Lys Cys Asn Gly Lys Asp
                   245                     250                     255
   gcg gtc acc aac ttc gat ccc agc acc tac gcc gag gag ttc gag ccg      816
   Ala Val Thr Asn Phe Asp Pro Ser Thr Tyr Ala Glu Glu Phe Glu Pro
               260                     265                     270
   gcg gct tcg acc ggc gac gcg gag cag cag aac ctg gac ctg tcg ctg      864
   Ala Ala Ser Thr Gly Asp Ala Glu Gln Gln Asn Leu Asp Leu Ser Leu
           275                     280                     285
   ggg agc tcg gcg ggg tcg aac aag agg ggc agc ctc gac ggc ggc ggc      912
   Gly Ser Ser Ala Gly Ser Asn Lys Arg Gly Ser Leu Asp Gly Gly Gly
       290                     295                     300
   atg gac gac gac ggc gcg gcg ggg tcc gac cag cgc gtc ccc atg gcc      960
   Met Asp Asp Asp Gly Ala Ala Gly Ser Asp Gln Arg Val Pro Met Ala
   305                 310                     315                 320
   ttc gag ctc gac tgg cac acg gcg gcg gcg cgc agc acc aag gcc aag     1008
   Phe Glu Leu Asp Trp His Thr Ala Ala Ala Arg Ser Thr Lys Ala Lys
                   325                     330                     335
   ttc gac cag aac tcg gcg cgt cat cag atg ccc cct cca gcc ctg caa     1056
   Phe Asp Gln Asn Ser Ala Arg His Gln Met Pro Pro Pro Ala Leu Gln
               340                 345                 350
   ctg caa gcc ccc cac atg cag ttc agt ccc agg cat cac caa ttc gtg      1104
   Leu Gln Ala Pro His Met Gln Phe Ser Pro Arg His His Gln Phe Val
           355                 360                 365
   ggc aac gcc gat ccg ggg aca gcg gga ggc ctg tcg ctg acg gtc ggc      1152
   Gly Asn Ala Asp Pro Gly Thr Ala Gly Gly Leu Ser Leu Thr Val Gly
       370                 375                 380
   gcc ggc gcc ggg ggc ggg caa tgg cct ccg cct ccg cct ccc cac cac      1200
   Ala Gly Ala Gly Gly Gly Gln Trp Pro Pro Pro Pro Pro Pro His His
   385                 390                 395                 400
   tac cag ccg ccg cat ccg cag cag cac cac cag cag cag caa cag agg      1248
   Tyr Gln Pro Pro His Pro Gln Gln His His Gln Gln Gln Gln Gln Arg
                   405                 410                 415
   ctg cag cac ggc tgg ggc aac gtc gtc ccc ggc acg agc tgg cag ccg      1296
   Leu Gln His Gly Trp Gly Asn Val Val Pro Gly Thr Ser Trp Gln Pro
               420                 425                 430
   gtc cag ccg ccg ccg ccg ccg cac cac cag gcg ggg ccg gcg ccg aac      1344
   Val Gln Pro Pro Pro Pro Pro His His Gln Ala Gly Pro Ala Pro Asn
           435                 440                 445
   aac gct gcc gcc gcc gca gca gca gcc gca gca tca tcc cga ttc cca      1392
   Asn Ala Ala Ala Ala Ala Ala Ala Ala Ala Ala Ser Ser Arg Phe Pro
       450                 455                 460
   ccc tac atc gcc acg cag gcg cag agc tgg ctc cag aag aac ggg ttc      1440
   Pro Tyr Ile Ala Thr Gln Ala Gln Ser Trp Leu Gln Lys Asn Gly Phe
   465                 470                 475                 480
   cac tcc ctg gcc aga ccc acc tag                                      1464
   His Ser Leu Ala Arg Pro Thr
                   485
<210> 5
   <211> 2691
   <212> DNA
   <213> Hordeum vulgare
<220>
   <221> exon
   <222> (1) .. (395)
<220>
   <221> Intron
   <222> (396) .. (668)
<220>
   <221> exon
   <222> (669) .. (694)
<220>
   <221> Intron
   <222> (695) .. (790)
<220>
   <221> exon
   <222> (791) .. (821)
<220>
   <221> Intron
   <222> (822) .. (916)
<220>
   <221> exon
   <222> (917) .. (1001)
<220>
   <221> Intron
   <222> (1002) .. (1083)
<220>
   <221> exon
   <222> (1084) .. (1229)
<220>
   <221> Intron
   <222> (1230) .. (1423)
<220>
   <221> exon
   <222> (1424) .. (1468)
<220>
   <221> Intron
   <222> (1469) .. (1600)
<220>
   <221> exon
   <222> (1601) .. (1691)
<220>
   <221> Intron
   <222> (1692) .. (1836)
<220>
   <221> exon
   <222> (1837) .. (2025)
<220>
   <221> Intron
   <222> (2026) .. (2132)
<220>
   <221> exon
   <222> (2133) .. (2222)
<220>
   <221> Intron
   <222> (2223) .. (2325)
<220>
   <221> exon
   <222> (2326) .. (2691)
<400> 5
   atg tgg gat ctc aac gac tcg ccg gcg gcc gag ggg ccg ccg ctg tcc        48
   Met Trp Asp Leu Asn Asp Ser Pro Ala Ala Glu Gly Pro Pro Leu Ser
   1               5                   10                  15
   ccg tcc gtg gac gac tcc ggc gcc tcc tcc tcg tct gcc gcc gcg gtg        96
   Pro Ser Val Asp Asp Ser Gly Ala Ser Ser Ser Ser Ala Ala Ala Val
               20                  25                  30
   gtc gag ata ccc gac gac gcc gag gac gac tcc gcc gag gcc gtc gtc       144
   Val Glu Ile Pro Asp Asp Ala Glu Asp Asp Ser Ala Glu Ala Val Val
           35                  40                  45
   acg cgc cag ttc ttc ccc ccg gcc gcc ccg ggc gag ggc ggc ccc ggc       192
   Thr Arg Gln Phe Phe Pro Pro Ala Ala Pro Gly Glu Gly Gly Pro Gly
       50                  55                  60
   aac ggg aac gac cgc gcc gcg tgg ctc cgc ctg gcc ggc gcc ccc gcg       240
   Asn Gly Asn Asp Arg Ala Ala Trp Leu Arg Leu Ala Gly Ala Pro Ala
   65                  70                  75                  80
   ccc acc gtg gcc gcg gcc gcc gga gga gga aca gga ggc ccc gcg gcg       288
   Pro Thr Val Ala Ala Ala Ala Gly Gly Gly Thr Gly Gly Pro Ala Ala
                   85                  90                  95
   gcg tcg gcg gcg gcc aag aag agc cgg cgc ggt ccc cgt tcc cgc agc       336
   Ala Ser Ala Ala Ala Lys Lys Ser Arg Arg Gly Pro Arg Ser Arg Ser
           100                   105                   110
   tcg cag tac cgc ggc gtc acc ttc tac cgc cgg acg ggc cgg tgg gag      384
   Ser Gln Tyr Arg Gly Val Thr Phe Tyr Arg Arg Thr Gly Arg Trp Glu
       115                   120                   125
   tcg cac ata tg  gtaagctccc gccgccgctc cgctcctttc ctttcctttc          435
   Ser His Ile Trp
       130
   tcgaaagctt aaggaaggaa ggagagagag tgcagcaaaa gaagagaaga gaagagaaga    495
   tggatggatg gatgggtttg gtattggatt cgtcttgttt tgggcattcc atgcctctgc    555
   tccttttacc tttcatagca acatcttatc aaacttattc cccttccttc cttgtttctc    615
   tctcttcccc cctctcctct cctcttctca ccccattctt ttgtgcaatg cag g gat     672
                                                                Asp
   tgc ggc aag cag gtc tat ctg g gtaagtttcc tccatccccg tcttgtccac       724
   Cys Gly Lys Gln Val Tyr Leu
       135                 140
   acatccatac ttggggtgcg aaatcgatcg ccaattggag ctcacaatgt cactgacggc    784
   ctgcag gt  gga ttc gac act gct cat gcg gcg gct cg  gtacgataaa        831
              Gly Gly Phe Asp Thr Ala His Ala Ala Ala Arg
                          145                 150
   gatctcatac caaccgattt gacagtacca atcaagctct cttctttact gatttcttct    891
   tcttcatcat catctgcggc cgcag g gcg tac gat cgg gcg gcg atc aag ttc    944
                                 Ala Tyr Asp Arg Ala Ala Ile Lys Phe
                                                 155                 160
   cgc ggc atg gag gcc gac atc aat ttc agc ctg gag gac tac gac gac      992
   Arg Gly Met Glu Ala Asp Ile Asn Phe Ser Leu Glu Asp Tyr Asp Asp
                   165                 170                 175
   atc aag cag gtgagcgacg cccgatcgat caacacgagc tagcgcttgt             1041
   Ile Lys Gln
   tgctcgcaga atcgtaacgt gtttgccctg gtttgcttgc ag atg ggc aac cta      1095
                                                  Met Gly Asn Leu
                                                  180
   acc aag gag gag ttc gtc cac gtg ctc cgg cgg cag agc acg ggg ttc     1143
   Thr Lys Glu Glu Phe Val His Val Leu Arg Arg Gln Ser Thr Gly Phe
       185                 190                 195
   ccc cgg ggg agc tcc aag tac agg ggc gtc acg ctc cac aag tgc ggc     1191
   Pro Arg Gly Ser Ser Lys Tyr Arg Gly Val Thr Leu His Lys Cys Gly
   200                 205                 210                 215
   agg tgg gag gcg cgg atg ggc cag ttc ctc ggc aag aa  gtatgtgctc      1239
   Arg Trp Glu Ala Arg Met Gly Gln Phe Leu Gly Lys Lys
                   220                 225
   ctccaccaac caccatcgcc accctccttc ctcccttctc ctccaatcga tggctcgaat   1299
   ttttgctctg ctcgtctgcc tgctcctgtc gttgcgctca aagttgcagc atctccctgc   1359
   taactagcct gacggcatgg catggcatgg cagcggcact aatgagaatc tttgtgcaac   1419
   gcag g tac gtc tac ttg ggg ctg ttc gat acc gag gag gaa gct gcc      1466
          Tyr Val Tyr Leu Gly Leu Phe Asp Thr Glu Glu Glu Ala Ala
              230                 235                 240
   ag  gtagtaaaaa ctgaaaaatt attgggcatg cactgtgcgc gcgtcctcca          1518
   Arg
   tccattgcta gctttagctt tgctcgggct ggggttgggc ggtgaactga ttgatgtctc   1578
   gtgtgtctct gtctctgggc ag g tcg tac gac cgc gct gcc atc aag tgc      1628
                              Ser Tyr Asp Arg Ala Ala Ile Lys Cys
                                  245                 250
   aac ggc aag gat gcg gtc acc aac ttc gat ccc agc acc tac gcc gag     1676
   Asn Gly Lys Asp Ala Val Thr Asn Phe Asp Pro Ser Thr Tyr Ala Glu
           255                 260                 265
   gag ttc gag ccg gcg ggtcagtaat aatcttgtta tattcatcgc gcactgttca    1731
   Glu Phe Glu Pro Ala
       270
   tctgtgattt tgatttggcc accataggat tatgcggcat acgtatgttt cttccggttc  1791
   gttcactgac cttgatcttg atcttgggtg ggcaatgctc tgcca gct tcg acc ggc  1848
                                                     Ala Ser Thr Gly
                                                         275
   gac gcg gag cag cag aac ctg gac ctg tcg ctg ggg agc tcg gcg ggg    1896
   Asp Ala Glu Gln Gln Asn Leu Asp Leu Ser Leu Gly Ser Ser Ala Gly
           280                 285                 290
   tcg aac aag agg ggc agc ctc gac ggc ggc ggc atg gac gac gac ggc    1944
   Ser Asn Lys Arg Gly Ser Leu Asp Gly Gly Gly Met Asp Asp Asp Gly
       295                 300                 305
   gcg gcg ggg tcc gac cag cgc gtc ccc atg gcc ttc gag ctc gac tgg    1992
   Ala Ala Gly Ser Asp Gln Arg Val Pro Met Ala Phe Glu Leu Asp Trp
   310                 315                 320                 325
   cac acg gcg gcg gcg cgc agc acc aag gcc aag gtacacgccc caacttgacc  2045
   His Thr Ala Ala Ala Arg Ser Thr Lys Ala Lys
                   330                 335
   ttgaccaccc tgcaacaaac tactgctcct gcatcatagt ataatcaagc gttggaaaag  2105
   ctgagaatgg cgtaatttac tatgcag ttc gac cag aac tcg gcg cgt cat cag  2159
                                 Phe Asp Gln Asn Ser Ala Arg His Gln
                                             340                 345
   atg ccc cct cca gcc ctg caa ctg caa gcc ccc cac atg cag ttc agt    2207
   Met Pro Pro Pro Ala Leu Gln Leu Gln Ala Pro His Met Gln Phe Ser
                   350                 355                 360
   ccc agg cat cac caa gtgggtactt ttgccgctcc aagttggagc tgacgaattt    2262
   Pro Arg His His Gln
               365
   ttctatctct tctcttcttg gtaggcgcga attaacggtg gttgtttggt tcaaactgtg  2322
   cag ttc gtg ggc aac gcc gat ccg ggg aca gcg gga ggc ctg tcg ctg    2370
       Phe Val Gly Asn Ala Asp Pro Gly Thr Ala Gly Gly Leu Ser Leu
                   370                 375                 380
   acg gtc ggc gcc ggc gcc ggg ggc ggg caa tgg cct ccg cct ccg cct    2418
   Thr Val Gly Ala Gly Ala Gly Gly Gly Gln Trp Pro Pro Pro Pro Pro
               385                 390                 395
   ccc cac cac tac cag ccg ccg cat ccg cag cag cac cac cag cag cag    2466
   Pro His His Tyr Gln Pro Pro His Pro Gln Gln His His Gln Gln Gln
           400                 405                 410
   caa cag agg ctg cag cac ggc tgg ggc aac gtc gtc ccc ggc acg agc    2514
   Gln Gln Arg Leu Gln His Gly Trp Gly Asn Val Val Pro Gly Thr Ser
       415                 420                 425
   tgg cag ccg gtc cag ccg ccg ccg ccg ccg cac cac cag gcg ggg ccg    2562
   Trp Gln Pro Val Gln Pro Pro Pro Pro Pro His His Gln Ala Gly Pro
   430                 435                 440                 445
   gcg ccg aac aac gct gcc gcc gcc gca gca gca gcc gca gca tca tcc    2610
   Ala Pro Asn Asn Ala Ala Ala Ala Ala Ala Ala Ala Ala Ala Ser Ser
                   450                 455                 460
   cga ttc cca ccc tac atc gcc acg cag gcg cag agc tgg ctc cag aag    2658
   Arg Phe Pro Pro Tyr Ile Ala Thr Gln Ala Gln Ser Trp Leu Gln Lys
               465                 470                 475
   aac ggg ttc cac tcc ctg gcc aga ccc acc tag                        2691
   Asn Gly Phe His Ser Leu Ala Arg Pro Thr
           480                 485
<210> 6
   <211> 487
   <212> PRT
   <213> Hordeum vulgare
<400> 6
   Met Trp Asp Leu Asn Asp Ser Pro Ala Ala Glu Gly Pro Pro Leu Ser
   1               5                   10                  15
   Pro Ser Val Asp Asp Ser Gly Ala Ser Ser Ser Ser Ala Ala Ala Val
               20                  25                  30
   Val Glu Ile Pro Asp Asp Ala Glu Asp Asp Ser Ala Glu Ala Val Val
           35                  40                  45
   Thr Arg Gln Phe Phe Pro Pro Ala Ala Pro Gly Glu Gly Gly Pro Gly
       50                  55                  60
   Asn Gly Asn Asp Arg Ala Ala Trp Leu Arg Leu Ala Gly Ala Pro Ala
   65                  70                  75                  80
   Pro Thr Val Ala Ala Ala Ala Gly Gly Gly Thr Gly Gly Pro Ala Ala
                   85                  90                  95
   Ala Ser Ala Ala Ala Lys Lys Ser Arg Arg Gly Pro Arg Ser Arg Ser
               100                 105                 110
   Ser Gln Tyr Arg Gly Val Thr Phe Tyr Arg Arg Thr Gly Arg Trp Glu
           115                 120                 125
   Ser His Ile Trp Asp Cys Gly Lys Gln Val Tyr Leu Gly Gly Phe Asp
       130                 135                 140
   Thr Ala His Ala Ala Ala Arg Ala Tyr Asp Arg Ala Ala Ile Lys Phe
   145                 150                 155                 160
   Arg Gly Met Glu Ala Asp Ile Asn Phe Ser Leu Glu Asp Tyr Asp Asp
                   165                 170                 175
   Ile Lys Gln Met Gly Asn Leu Thr Lys Glu Glu Phe Val His Val Leu
               180                 185                 190
   Arg Arg Gln Ser Thr Gly Phe Pro Arg Gly Ser Ser Lys Tyr Arg Gly
           195                 200                 205
   Val Thr Leu His Lys Cys Gly Arg Trp Glu Ala Arg Met Gly Gln Phe
       210                 215                 220
   Leu Gly Lys Lys Tyr Val Tyr Leu Gly Leu Phe Asp Thr Glu Glu Glu
   225                 230                 235                 240
   Ala Ala Arg Ser Tyr Asp Arg Ala Ala Ile Lys Cys Asn Gly Lys Asp
                   245                 250                 255
   Ala Val Thr Asn Phe Asp Pro Ser Thr Tyr Ala Glu Glu Phe Glu Pro
               260                 265                 270
   Ala Ala Ser Thr Gly Asp Ala Glu Gln Gln Asn Leu Asp Leu Ser Leu
           275                 280                 285
   Gly Ser Ser Ala Gly Ser Asn Lys Arg Gly Ser Leu Asp Gly Gly Gly
       290                 295                 300
   Met Asp Asp Asp Gly Ala Ala Gly Ser Asp Gln Arg Val Pro Met Ala
   305                 310                 315                 320
   Phe Glu Leu Asp Trp His Thr Ala Ala Ala Arg Ser Thr Lys Ala Lys
                   325                 330                 335
   Phe Asp Gln Asn Ser Ala Arg His Gln Met Pro Pro Pro Ala Leu Gln
               340                 345                 350
   Leu Gln Ala Pro His Met Gln Phe Ser Pro Arg His His Gln Phe Val
           355                 360                 365
   Gly Asn Ala Asp Pro Gly Thr Ala Gly Gly Leu Ser Leu Thr Val Gly
       370                 375                 380
   Ala Gly Ala Gly Gly Gly Gln Trp Pro Pro Pro Pro Pro Pro His His
   385                 390                 395                 400
   Tyr Gln Pro Pro His Pro Gln Gln His His Gln Gln Gln Gln Gln Arg
   405 410 415
   Leu Gln His Gly Trp Gly Asn Val Val Pro Gly Thr Ser Trp Gln Pro
   420 425 430
   Val Gln Pro Pro Pro Pro Pro His His Gln Ala Gly Pro Ala Pro Asn
   435 440 445
   Asn Ala Ala Ala Ala Ala Ala Ala Ala Ala Ala Ser Ser Arg Phe Pro
   450 455 460
   Pro Tyr Ile Ala Thr Gln Ala Gln Ser Trp Leu Gln Lys Asn Gly Phe
   465 470 475 480
   His Ser Leu Ala Arg Pro Thr
   485
<210> 7
   <211> 1464
   <212> DNA
   <213> Hordeum vulgare
<220>
   <221> CDS
   <222> (1)..(1461)
   <223>
<400> 7
   atg tgg gat ctc aac gac tcg ccg gcg gcc gag ggg ccg ccg ctg tcc        48
   Met Trp Asp Leu Asn Asp Ser Pro Ala Ala Glu Gly Pro Pro Leu Ser
   1               5                   10                  15
   ccg tcc gtg gac gac tcc ggc gcc tcc tcc tcg tct gcc gcc gcg gtg        96
   Pro Ser Val Asp Asp Ser Gly Ala Ser Ser Ser Ser Ala Ala Ala Val
               20                  25                  30
   gtc gag ata ccg gac gac gcc gag gac gac tcc gcc gag gcc gtc gtc       144
   Val Glu Ile Pro Asp Asp Ala Glu Asp Asp Ser Ala Glu Ala Val Val
           35                  40                  45
   acg cgc cag ttc ttc ccc ccg gcc gcc ccg ggc gag ggc ggc ccc ggc       192
   Thr Arg Gln Phe Phe Pro Pro Ala Ala Pro Gly Glu Gly Gly Pro Gly
       50                  55                  60
   aac ggg aac gac cgc gcc gcg tgg ctc cgc ctg gcc ggc gcc ccc gcg       240
   Asn Gly Asn Asp Arg Ala Ala Trp Leu Arg Leu Ala Gly Ala Pro Ala
   65                  70                  75                  80
   ccc acc gtg gcc gcg gcc gcc gga gga gga aca gga ggc ccc gcg gcg       288
   Pro Thr Val Ala Ala Ala Ala Gly Gly Gly Thr Gly Gly Pro Ala Ala
                   85                  90                  95
   gcg tcg gcg gcg gcc aag aag agc cgg cgc ggt ccc cgt tcc cgc agc       336
   Ala Ser Ala Ala Ala Lys Lys Ser Arg Arg Gly Pro Arg Ser Arg Ser
               100                 105                 110
   tcg cag tac cgc ggc gtc acc ttc tac cgc cgg acg ggc cgg tgg gag       384
   Ser Gln Tyr Arg Gly Val Thr Phe Tyr Arg Arg Thr Gly Arg Trp Glu
           115                 120                 125
   tcg cac ata tgg gat tgc ggc aag cag gtc tat ctg ggt gga ttc gac       432
   Ser His Ile Trp Asp Cys Gly Lys Gln Val Tyr Leu Gly Gly Phe Asp
       130                 135                 140
   act gct cat gcg gcg gct cgg gcg tac gat cgg gcg gcg atc aag ttc       480
   Thr Ala His Ala Ala Ala Arg Ala Tyr Asp Arg Ala Ala Ile Lys Phe
   145                 150                 155                 160
   cgc ggc atg gag gcc gac atc aat ttc agc ctg gag gac tac gac gac       528
   Arg Gly Met Glu Ala Asp Ile Asn Phe Ser Leu Glu Asp Tyr Asp Asp
                   165                 170                 175
   atc aag cag atg ggc aac ctg acc aag gag gag ttc gtc cac gtg ctc       576
   Ile Lys Gln Met Gly Asn Leu Thr Lys Glu Glu Phe Val His Val Leu
               180                 185                 190
   cgg cgg cag agc acg ggg ttc ccc cgg ggg agc tcc aag tac agg ggc      624
   Arg Arg Gln Ser Thr Gly Phe Pro Arg Gly Ser Ser Lys Tyr Arg Gly
           195                 200                 205
   gtc acg ctc cac aag tgc ggc agg tgg gag gcg cgg atg ggc cag ttc      672
   Val Thr Leu His Lys Cys Gly Arg Trp Glu Ala Arg Met Gly Gln Phe
       210                 215                 220
   ctc ggc aag aag tac gtc tac ttg ggg ctg ttc gat acc gag gag gaa      720
   Leu Gly Lys Lys Tyr Val Tyr Leu Gly Leu Phe Asp Thr Glu Glu Glu
   225                 230                 235                 240
   gct gcc agg tcg tac gac cgc gct gcc atc aag tgc aac ggc aag gat      768
   Ala Ala Arg Ser Tyr Asp Arg Ala Ala Ile Lys Cys Asn Gly Lys Asp
                   245                 250                 255
   gcg gtc acc aac ttc gat ccc agc acc tac gcc gag gag ttc gag ccg      816
   Ala Val Thr Asn Phe Asp Pro Ser Thr Tyr Ala Glu Glu Phe Glu Pro
               260                 265                 270
   gcg gct tcg acc ggc gac gcg gag cag cag aac ctg gac ctg tcg ctg      864
   Ala Ala Ser Thr Gly Asp Ala Glu Gln Gln Asn Leu Asp Leu Ser Leu
           275                 280                 285
   ggg agc tcg gcg ggg tcg aac aag agg ggc agc ctc gac ggc ggc ggc      912
   Gly Ser Ser Ala Gly Ser Asn Lys Arg Gly Ser Leu Asp Gly Gly Gly
       290                 295                 300
   atg gac gac gac ggc gcg gcg ggg tcc gac cag cgc gtc ccc atg gcc      960
   Met Asp Asp Asp Gly Ala Ala Gly Ser Asp Gln Arg Val Pro Met Ala
   305                 310                 315                 320
   ttc gag ctc gac tgg cac acg gcg gcg gcg cgc agc acc aag gcc aag     1008
   Phe Glu Leu Asp Trp His Thr Ala Ala Ala Arg Ser Thr Lys Ala Lys
                   325                 330                 335
   ttc gac cag aac tcg gcg cgt cat cag atg ccc cct cca gcc ctg caa     1056
   Phe Asp Gln Asn Ser Ala Arg His Gln Met Pro Pro Pro Ala Leu Gln
               340                 345                 350
   ctg caa gcc ccc cac atg cag ttc agt ccc agg cat cac caa ttc gtg     1104
   Leu Gln Ala Pro His Met Gln Phe Ser Pro Arg His His Gln Phe Val
           355                 360                 365
   ggc aac gcc gat ccg ggg aca gcg gga ggc ctg tcg ctg acg gtc ggc     1152
   Gly Asn Ala Asp Pro Gly Thr Ala Gly Gly Leu Ser Leu Thr Val Gly
       370                 375                 380
   gcc ggc gcc ggg ggc ggg caa tgg cct ccg cct ccg cct ccc cac cac     1200
   Ala Gly Ala Gly Gly Gly Gln Trp Pro Pro Pro Pro Pro Pro His His
   385                 390                 395                 400
   tac cag ccg ccg cat ccg cag cag cac cac cag cag cag caa cag agg     1248
   Tyr Gln Pro Pro His Pro Gln Gln His His Gln Gln Gln Gln Gln Arg
                   405                 410                 415
   ctg cag cac ggc tgg ggc aac gtc gtc ccc ggc acg agc tgg cag ccg     1296
   Leu Gln His Gly Trp Gly Asn Val Val Pro Gly Thr Ser Trp Gln Pro
               420                 425                 430
   gtc cag ccg ccg ccg ccg ccg cac cac cag gcg ggg ccg gcg ccg aac     1344
   Val Gln Pro Pro Pro Pro Pro His His Gln Ala Gly Pro Ala Pro Asn
           435                 440                 445
   aac gct gcc gcc gcc gca gca gca gca gca gca tca tca cga ttc cca     1392
   Asn Ala Ala Ala Ala Ala Ala Ala Ala Ala Ala Ser Ser Arg Phe Pro
       450                 455                 460
   ccc tac atc gcc acg cag gcg cag agc tgg ctc cag aag aac ggg ttc     1440
   Pro Tyr Ile Ala Thr Gln Ala Gln Ser Trp Leu Gln Lys Asn Gly Phe
   465                 470                 475                 480
   cac tcc ctg gcc aga ccc acc tag                                   1464
   His Ser Leu Ala Arg Pro Thr
                   485
<210> 8
   <211> 2691
   <212> DNA
   <213> Hordeum vulgare
<220>
   <221> exon
   <222> (1)..(395)
<220>
   <221> Intron
   <222> (396)..(668)
<220>
   <221> exon
   <222> (669)..(694)
<220>
   <221> Intron
   <222> (695)..(790)
<220>
   <221> exon
   <222> (791)..(821)
<220>
   <221> Intron
   <222> (822)..(916)
<220>
   <221> exon
   <222> (917)..(1001)
<220>
   <221> Intron
   <222> (1002)..(1083)
<220>
   <221> exon
   <222> (1084)..(1229)
<220>
   <221> Intron
   <222> (1230)..(1423)
<220>
   <221> exon
   <222> (1424)..(1468)
<220>
   <221> Intron
   <222> (1469)..(1600)
<220>
   <221> exon
   <222> (1601)..(1691)
<220>
   <221> Intron
   <222> (1692)..(1836)
<220>
   <221> exon
   <222> (1837)..(2025)
<220>
   <221> Intron
   <222> (2026)..(2132)
<220>
   <221> exon
   <222> (2133)..(2222)
<220>
   <221> Intron
   <222> (2223)..(2325)
<220>
   <221> exon
   <222> (2326)..(2691)
<400> 8
   atg tgg gat ctc aac gac tcg ccg gcg gcc gag ggg ccg ccg ctg tcc      48
   Met Trp Asp Leu Asn Asp Ser Pro Ala Ala Glu Gly Pro Pro Leu Ser
   1               5                   10                  15
   ccg tcc gtg gac gac tcc ggc gcc tcc tcc tcg tct gcc gcc gcg gtg      96
   Pro Ser Val Asp Asp Ser Gly Ala Ser Ser Ser Ser Ala Ala Ala Val
               20                  25                  30
   gtc gag ata ccg gac gac gcc gag gac gac tcc gcc gag gcc gtc gtc      144
   Val Glu Ile Pro Asp Asp Ala Glu Asp Asp Ser Ala Glu Ala Val Val
           35                  40                  45
   acg cgc cag ttc ttc ccc ccg gcc gcc ccg ggc gag ggc ggc ccc ggc      192
   Thr Arg Gln Phe Phe Pro Pro Ala Ala Pro Gly Glu Gly Gly Pro Gly
       50                  55                  60
   aac ggg aac gac cgc gcc gcg tgg ctc cgc ctg gcc ggc gcc ccc gcg      240
   Asn Gly Asn Asp Arg Ala Ala Trp Leu Arg Leu Ala Gly Ala Pro Ala
   65                  70                  75                  80
   ccc acc gtg gcc gcg gcc gcc gga gga gga aca gga ggc ccc gcg gcg      288
   Pro Thr Val Ala Ala Ala Ala Gly Gly Gly Thr Gly Gly Pro Ala Ala
                   85                  90                  95
   gcg tcg gcg gcg gcc aag aag agc cgg cgc ggt ccc cgt tcc cgc agc      336
   Ala Ser Ala Ala Ala Lys Lys Ser Arg Arg Gly Pro Arg Ser Arg Ser
               100                 105                 110
   tcg cag tac cgc ggc gtc acc ttc tac cgc cgg acg ggc cgg tgg gag      384
   Ser Gln Tyr Arg Gly Val Thr Phe Tyr Arg Arg Thr Gly Arg Trp Glu
           115                 120                 125
   tcg cac ata tg  gtaagctccc gccgccgctc cgctcctttc ctttcctttc          435
   Ser His Ile Trp
       130
   tcgaaagctt aaggaaggaa ggagagagag tgcagcaaaa gaagagaaga gaagagaaga    495
   tggatggatg gatgggtttg gtattggatt cgtcttgttt tgggcattcc atgcctctgc    555
   tccttttacc tttcatagca acatcttatc aaacttattc cccttccttc cttgtttctc    615
   tctcttcccc cctctcctct cctcttctca ccccattctt ttgtgcaatg cag g gat     672
                                                                Asp
   tgc ggc aag cag gtc tat ctg g gtaagtttcc tccatccccg tcttgtccac       724
   Cys Gly Lys Gln Val Tyr Leu
       135                 140
   acatccatac ttggggtgcg aaatcgatcg ccaattggag ctcacaatgt cactgacggc    784
   ctgcag gt  gga ttc gac act gct cat gcg gcg gct cg  gtacgataaa       831
          Gly Gly Phe Asp Thr Ala His Ala Ala Ala Arg
                      145                 150
   gatctcatac caaccgattt gacagtacca atcaagctct cttctttact gatttcttct    891
   tcttcatcat catctgcggc cgcag g gcg tac gat cgg gcg gcg atc aag ttc    944
                                 Ala Tyr Asp Arg Ala Ala Ile Lys Phe
                                         155                 160
   cgc ggc atg gag gcc gac atc aat ttc agc ctg gag gac tac gac gac      992
   Arg Gly Met Glu Ala Asp Ile Asn Phe Ser Leu Glu Asp Tyr Asp Asp
                   165                 170                 175
   atc aag cag gtgagcgacg cccgatcgat caacacgagc tagcgcttgt             1041
   Ile Lys Gln
   tgctcgcaga atcgtaacgt gtttgccctg gtttgcttgc ag atg ggc aac ctg      1095
                                                   Met Gly Asn Leu
                                                   180
   acc aag gag gag ttc gtc cac gtg ctc cgg cgg cag agc acg ggc ttc     1143
   Thr Lys Glu Glu Phe Val His Val Leu Arg Arg Gln Ser Thr Gly Phe
       185                 190                 195
   ccc cgg ggg agc tcc aag tac agg ggc gtc acg ctc cac aag tgc ggc     1191
   Pro Arg Gly Ser Ser Lys Tyr Arg Gly Val Thr Leu His Lys Cys Gly
   200                 205                 210                 215
   agg tgg gag gcg cgg atg ggc cag ttc ctc ggc aag aa  gtatgtgctc      1239
   Arg Trp Glu Ala Arg Met Gly Gln Phe Leu Gly Lys Lys
                   220                 225
   ctccaccaac caccatcgcc accctccttc ctcccttctc ctccaatcga tggctcgaat   1299
   ttttgctctg ctcgtctgcc tgctcctgtc gttgcgctca aagttgcagc atctccctgc   1359
   taactagcct gacggcatgg catggcatgg cagcggcact aatgagaatc tttgtgcaac   1419
   gcag g tac gtc tac ttg ggg ctg ttc gat acc gag gag gaa gct gcc      1466
          Tyr Val Tyr Leu Gly Leu Phe Asp Thr Glu Glu Glu Ala Ala
              230                 235                 240
   ag  gtagtaaaaa ctgaaaaatt attgggcatg cactgtgcgc gcgtcctcca          1518
   Arg
   tccattgcta gctttagctt tgctcgggct ggggttgggc ggtgaactga ttgatgtctc   1578
   gtgtgtctct gtctctgggc ag g tcg tac gac cgc gct gcc atc aag tgc      1628
                            Ser Tyr Asp Arg Ala Ala Ile Lys Cys
                                245                 250
   aac ggc aag gat gcg gtc acc aac ttc gat ccc agc acc tac gcc gag     1676
   Asn Gly Lys Asp Ala Val Thr Asn Phe Asp Pro Ser Thr Tyr Ala Glu
           255                 260                 265
   gag ttc gag ccg gcg ggtcagtaat aatcttgtta tattcatcgc gcactgttca     1731
   Glu Phe Glu Pro Ala
       270
   tctgtgattt tgatttggcc accataggat tatgcggcat acgtatgttt cttccggttc   1791
   gttcactgac cttgatcttg atcttgggtg ggcaatgctc tgcca gct tcg acc ggc   1848
                                                      Ala Ser Thr Gly
                                                          275
   gac gcg gag cag cag aac ctg gac ctg tcg ctg ggg agc tcg gcg ggg     1896
   Asp Ala Glu Gln Gln Asn Leu Asp Leu Ser Leu Gly Ser Ser Ala Gly
           280                 285                 290
   tcg aac aag agg ggc agc ctc gac ggc ggc ggc atg gac gac gac ggc     1944
   Ser Asn Lys Arg Gly Ser Leu Asp Gly Gly Gly Met Asp Asp Asp Gly
       295                 300                 305
   gcg gcg ggg tcc gac cag cgc gtc ccc atg gcc ttc gag ctc gac tgg     1992
   Ala Ala Gly Ser Asp Gln Arg Val Pro Met Ala Phe Glu Leu Asp Trp
   310                 315                 320                 325
   cac acg gcg gcg gcg cgc agc acc aag gcc aag gtacacgccc caacttaacc   2045
   His Thr Ala Ala Ala Arg Ser Thr Lys Ala Lys
                   330                 335
   ttgaccaccc tgcaacaaac tactgctcct gcatcatagt ataatcaagc gttggaaaag   2105
   ctgagaatgg cgtaatttac tatgcag ttc gac cag aac tcg gcg cgt cat cag   2159
                                 Phe Asp Gln Asn Ser Ala Arg His Gln
                                                 340                 345
   atg ccc cct cca gcc ctg caa ctg caa gcc ccc cac atg cag ttc agt     2207
   Met Pro Pro Pro Ala Leu Gln Leu Gln Ala Pro His Met Gln Phe Ser
                   350                 355                 360
   ccc agg cat cac caa gtgggtactt ttgccgctcc aagttggagc tcacgaattt      2262
   Pro Arg His His Gln
               365
   ttctatctct tctcttcttg gtaggcgcga attaacggtg gttgtttggt tcaaactgtg   2322
   cag ttc gtg ggc aac gcc gat ccg ggg aca gcg gga ggc ctg tcg ctg      2370
       Phe Val Gly Asn Ala Asp Pro Gly Thr Ala Gly Gly Leu Ser Leu
                   370                 375                 380
   acg gtc ggc gcc ggc gcc ggg ggc ggg caa tgg cct ccg cct ccg cct      2418
   Thr Val Gly Ala Gly Ala Gly Gly Gly Gln Trp Pro Pro Pro Pro Pro
               385                 390                 395
   ccc cac cac tac cag ccg ccg cat ccg cag cag cac cac cag cag cag      2466
   Pro His His Tyr Gln Pro Pro His Pro Gln Gln His His Gln Gln Gln
           400                 405                 410
   caa cag agg ctg cag cac ggc tgg ggc aac gtc gtc ccc ggc acg agc      2514
   Gln Gln Arg Leu Gln His Gly Trp Gly Asn Val Val Pro Gly Thr Ser
       415                 420                 425
   tgg cag ccg gtc cag ccg ccg ccg ccg ccg cac cac cag gcg ggg ccg      2562
   Trp Gln Pro Val Gln Pro Pro Pro Pro Pro His His Gln Ala Gly Pro
   430                 435                 440                 445
   gcg ccg aac aac gct gcc gcc gcc gca gca gca gca gca gca tca tca      2610
   Ala Pro Asn Asn Ala Ala Ala Ala Ala Ala Ala Ala Ala Ala Ser Ser
                   450                 455                 460
   cga ttc cca ccc tac atc gcc acg cag gcg cag agc tgg ctc cag aag      2658
   Arg Phe Pro Pro Tyr Ile Ala Thr Gln Ala Gln Ser Trp Leu Gln Lys
               465                 470                 475
   aac ggg ttc cac tcc ctg gcc aga ccc acc tag                          2691
   Asn Gly Phe His Ser Leu Ala Arg Pro Thr
           480                 485
<210> 9
   <211> 487
   <212> PRT
   <213> Hordeum vulgare
<400> 9
   Met Trp Asp Leu Asn Asp Ser Pro Ala Ala Glu Gly Pro Pro Leu Ser
   1               5                   10                  15
   Pro Ser Val Asp Asp Ser Gly Ala Ser Ser Ser Ser Ala Ala Ala Val
               20                  25                  30
   Val Glu Ile Pro Asp Asp Ala Glu Asp Asp Ser Ala Glu Ala Val Val
           35                  40                  45
   Thr Arg Gln Phe Phe Pro Pro Ala Ala Pro Gly Glu Gly Gly Pro Gly
       50                  55                  60
   Asn Gly Asn Asp Arg Ala Ala Trp Leu Arg Leu Ala Gly Ala Pro Ala
   65                  70                  75                  80
   Pro Thr Val Ala Ala Ala Ala Gly Gly Gly Thr Gly Gly Pro Ala Ala
                   85                  90                  95
   Ala Ser Ala Ala Ala Lys Lys Ser Arg Arg Gly Pro Arg Ser Arg Ser
               100                 105                 110
   Ser Gln Tyr Arg Gly Val Thr Phe Tyr Arg Arg Thr Gly Arg Trp Glu
           115                 120                 125
   Ser His Ile Trp Asp Cys Gly Lys Gln Val Tyr Leu Gly Gly Phe Asp
       130                 135                 140
   Thr Ala His Ala Ala Ala Arg Ala Tyr Asp Arg Ala Ala Ile Lys Phe
   145                 150                 155                 160
   Arg Gly Met Glu Ala Asp Ile Asn Phe Ser Leu Glu Asp Tyr Asp Asp
   165 170 175
   Ile Lys Gln Met Gly Asn Leu Thr Lys Glu Glu Phe Val His Val Leu
   180 185 190
   Arg Arg Gln Ser Thr Gly Phe Pro Arg Gly Ser Ser Lys Tyr Arg Gly
   195 200 205
   Val Thr Leu His Lys Cys Gly Arg Trp Glu Ala Arg Met Gly Gln Phe
   210 215 220
   Leu Gly Lys Lys Tyr Val Tyr Leu Gly Leu Phe Asp Thr Glu Glu Glu
   225 230 235 240
   Ala Ala Arg Ser Tyr Asp Arg Ala Ala Ile Lys Cys Asn Gly Lys Asp
   245 250 255
   Ala Val Thr Asn Phe Asp Pro Ser Thr Tyr Ala Glu Glu Phe Glu Pro
   260 265 270
   Ala Ala Ser Thr Gly Asp Ala Glu Gln Gln Asn Leu Asp Leu Ser Leu
   275 280 285
   Gly Ser Ser Ala Gly Ser Asn Lys Arg Gly Ser Leu Asp Gly Gly Gly
   290 295 300
   Met Asp Asp Asp Gly Ala Ala Gly Ser Asp Gln Arg Val Pro Met Ala
   305 310 315 320
   Phe Glu Leu Asp Trp His Thr Ala Ala Ala Arg Ser Thr Lys Ala Lys
   325 330 335
   Phe Asp Gln Asn Ser Ala Arg His Gln Met Pro Pro Pro Ala Leu Gln
   340 345 350
   Leu Gln Ala Pro His Met Gln Phe Ser Pro Arg His His Gln Phe Val
   355 360 365
   Gly Asn Ala Asp Pro Gly Thr Ala Gly Gly Leu Ser Leu Thr Val Gly
   370 375 380
   Ala Gly Ala Gly Gly Gly Gln Trp Pro Pro Pro Pro Pro Pro His His
   385 390 395 400
   Tyr Gln Pro Pro His Pro Gln Gln His His Gln Gln Gln Gln Gln Arg
   405 410 415
   Leu Gln His Gly Trp Gly Asn Val Val Pro Gly Thr Ser Trp Gln Pro
   420 425 430
   Val Gln Pro Pro Pro Pro Pro His His Gln Ala Gly Pro Ala Pro Asn
   435 440 445
   Asn Ala Ala Ala Ala Ala Ala Ala Ala Ala Ala Ser Ser Arg Phe Pro
   450 455 460
   Pro Tyr Ile Ala Thr Gln Ala Gln Ser Trp Leu Gln Lys Asn Gly Phe
   465 470 475 480
   His Ser Leu Ala Arg Pro Thr
   485
<210> 10
   <211 > 21
   <212> RNA
   <213> Oryza sativa
<220>
   <221> misc_RNA
   <222> (1)..(21)
   <223> micro RNA
<400> 10
   ggaaucuuga ugaugcugca u 21
<210> 11
   <211> 21
   <212> DNA
   <213> Hordeum vulgare
<220>
   <221> misc_feature
   <222> (1)..(21)
   <223> micro RNA targeting site
<400> 11
   cagcagcatc atcacgattc c 21
<210> 12
   <211> 21
   <212> DNA
   <213> Artificial
<220>
   <223> An artificially synthesized primer sequence
<400> 12
   accagcagca gcaacagagg c 21
<210> 13
   <211> 21
   <212> DNA
   <213> Artificial
<220>
   <223> An artificially synthesized primer sequence
<400> 13
   gctggtaatg gctgtgggac g 21
<210> 14
   <211> 21
   <212> DNA
   <213> Artificial
<220>
   <223> An artificially synthesized primer sequence
<400> 14
   tcaagagcag ccagccaaga a 21
<210> 15
   <211> 21
   <212> DNA
   <213> Artificial
<220>
   <223> An artificially synthesized primer sequence
<400> 15
   ccgcatccgt cctcctctca a 21
<210> 16
   <211> 21
   <212> DNA
   <213> Artificial
<220>
   <223> An artificially synthesized primer sequence
<400> 16
   tccatctctc gctctcaccc a 21

## Description

### [Technical Field]

The present invention relates to a gene controlling chasmogamy/cleistogamy of a plant, as well as relates to determination of chasmogamy/cleistogamy of a plant and production of a cleistogamous plant using the gene.

### [Background Art]

World's important cereals such as rice, wheat, and barley are all crop plants whose seeds (embryos and albumens) are eaten, and are each a self-pollinating crop plant which produces seeds in such a manner that a stamen and a pistil develop in each single flower, and undergo self-pollination. Flowers of these crop plants generally open at pollination, and anthers of the flowers generally project to the outside (chasmogamy). However, it is known that, since self-pollination inside the flowers is possible and open-flowering is not necessarily required for fructification, flowers of these crop plants may undergo closed-flower pollination (cleistogamy), where pollination occurs without opening of the flowers, depending on environmental conditions (NPL1). Meanwhile, it has been known from the past that some barley varieties do not open their flowers, and undergo closed-flower pollination, genetically (NPL 2).

Recently, the cleistogamy of barley has been shown to be associated with a gene effective for improvement of resistance to Fusarium ear blight of barley, and it has been revealed that introduction of cleistogamy is extremely effective means for preventing infection of barley with Fusarium ear blight (NPL 3). Fusarium ear blight is the most important disease among plants of the subfamily Pooideae, and the enhancement of resistance against Fusarium ear blight has been sought. Accordingly, the introducing of cleistogamy is necessary for quality improvement of barley.

Conventional breeding of a crop plant has required a large farm field, a lot of man power, and a considerable period, because it is necessary to mate a cultivar, a wild species, or the like having a target trait, actually raise many individuals, select individuals having the target trait, and genetically fix the target trait. For example, when the target trait is chasmogamy/cleistogamy, it is necessary to specify individuals to be selected, by raising a selection target population, and investigating at anthesis whether each individual is chasmogamous or cleistogamous.

In this respect, breeding methods based on selection using a genetic marker as an index have been used recently in order to shorten the breeding period and to reduce manpower and the farm field area. The breeding based on such a genetic marker enables easy estimation about the presence or absence of the target trait on the basis of the genotype of the marker, and hence enables selection at a seed or seedling stage. Breeding of a cleistogamous variety using a marker is proposed also for barley (PTLs 1 and 2).

However, the breeding of a cleistogamous variety using a marker is inferior in specificity (accuracy) to the breeding using a gene controlling the target trait itself as a target. Hence, it is necessary to identify a gene controlling the chasmogamy/cleistogamous trait in order to conduct more efficient breeding.

For this reason, the identifying of a gene controlling the closed-flower pollination character of barley has been attempted over years, but the gene is yet to be identified, for example, because genomic analysis information on barley is insufficient.

### [Citation List]

### [Patent Literature]

[PTL 1] Japanese Unexamined Patent Application Publication No. 2004-180570
[PTL 2] Japanese Unexamined Patent Application Publication No. 2005-229854

### [Non Patent Literature]

[NPL 1] Hoshikawa Kiyochika, "The Growing Rice Plant", Rural Culture Association Japan, 1975, p 249 to 252
[NPL 2] Briggs D. E., "Barley", Chapman and Hall, London, ISBN: 041211870X, 1978, p. 45
[NPL 3] Yoshida Megumi, Kawada Naoyuki, Tohnooka Takuji, Breeding Research 4 (Suppl. 2), p. 303, (2002), (proceedings of 102nd Meeting of Japanese Society of Breeding; Japanese Society of Breeding)

### [Summary of Invention]

### [Technical Problem]

The present invention has been made in view of such circumstances, and an object of the present invention is to identify a novel gene controlling chasmogamy/cleistogamy of a plant such as barley, and elucidate the mechanism of development of chasmogamy/cleistogamy. Moreover, another object of the present invention is to provide a method for efficiently determining chasmogamy/cleistogamy of a plant on the basis of the elucidated mechanism of development of chasmogamy/cleistogamy. Still another object of the present invention is to provide a method for efficiently producing a plant having a closed-flower pollination trait on the basis of the elucidated mechanism of development of chasmogamy/cleistogamy.

### [Solution to Problem]

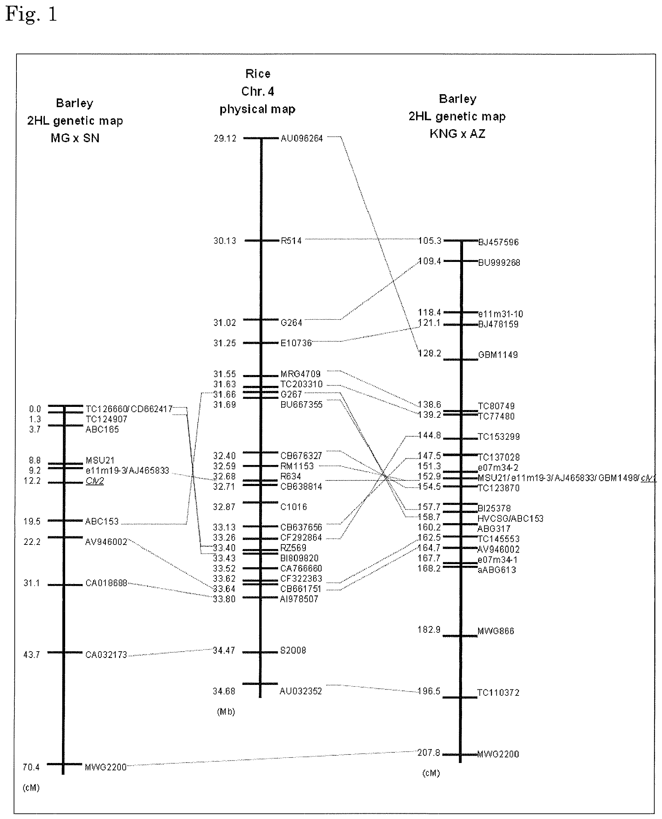

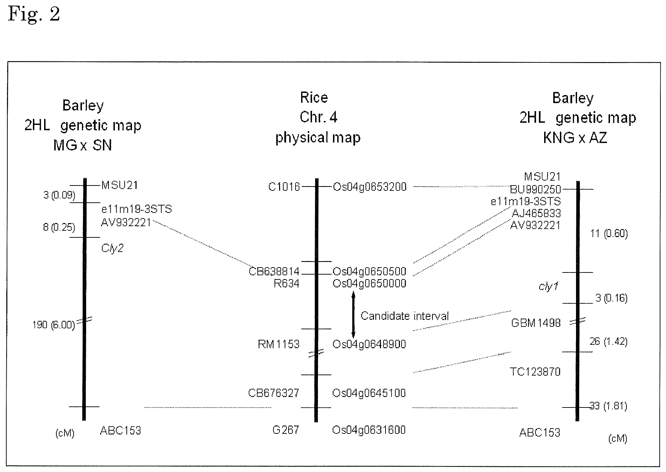

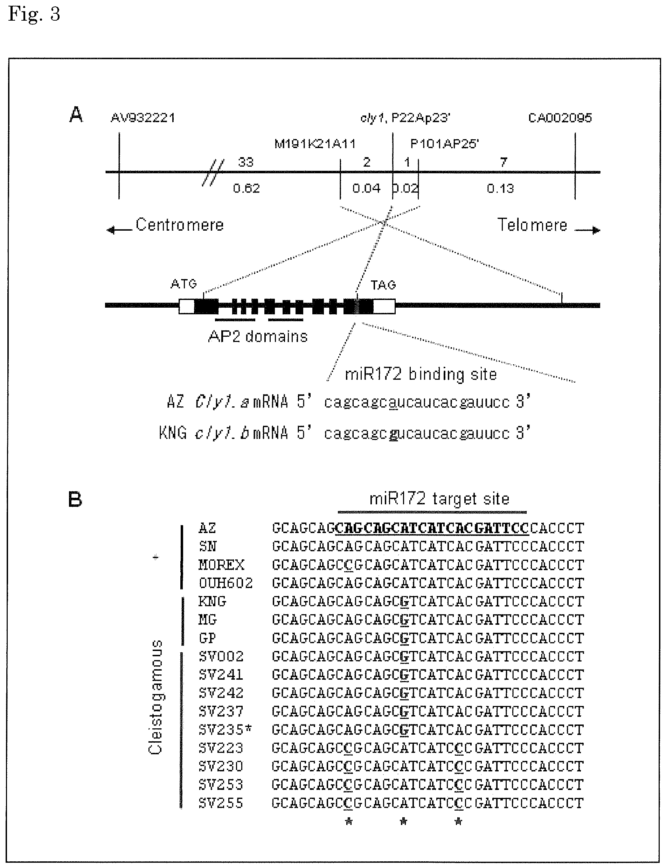

To achieve the above-described objects, the present inventors have attempted to identify a gene controlling chasmogamy/cleistogamy of barley by a positional cloning approach. Here, the situation was that it was extremely difficult to identify a target gene from only the known genomic information regarding barley, because the entire genomic base sequence was not determined for barley. Hence, the present inventors first built, by a unique approach, comparative genetic maps between chromosome 4 of rice and chromosome 2H of barley (Patent Document 1) in which the presence of the target gene was suggested (Fig. 1). Subsequently, by use of markers on the genetic map, an analysis was conducted on a large-scale F2 segregation population of a chasmogamous barley variety "Azumamugi" and a cleistogamous barley variety "Kanto Nakate Gold." Thus, a candidate region in which the cleistogamy gene cly1 seemed to be present was narrowed to a region of approximately 0.7 cM (Fig. 2 and Fig. 3A). The approximately 0.7-cM genome region corresponded to a 90-kb genome region of rice (Fig. 2 and Fig. 3A). The present inventors found 11 genes within the genomic region of rice, and presumed that, of these genes, a gene encoding a transcription factor having two AP2 domains was a gene corresponding to a chasmogamy gene Cly1 of barley.

EST base sequences partially encoding the barley gene corresponding to the AP2 gene of rice were extracted from databases, and a BAC library of a chasmogamous variety Morex was screened by use of markers created based on the sequence information. The base sequences of selected BAC clones were determined, and new markers were prepared based on the information concerning the base sequences. Then, by use of the markers, a large-scale F2 segregation population analysis was conducted. As a result, the candidate region in which cly1 seemed to be present was narrowed to a region of approximately 7 kb (Fig. 3A). Since any gene other than the AP2 gene was present in the 7-kb region, it was supported that cly1 encoded AP2.

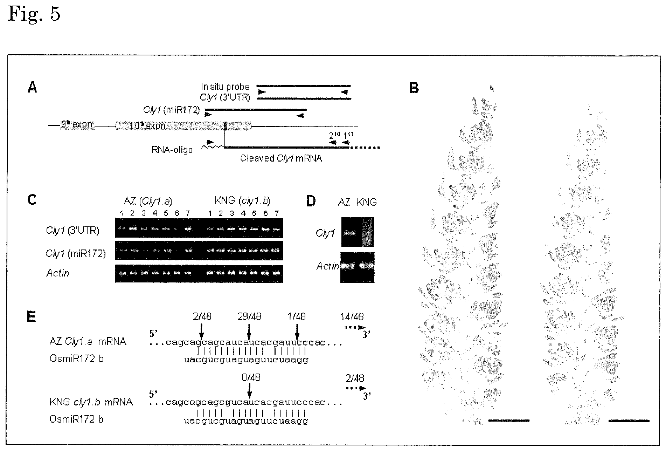

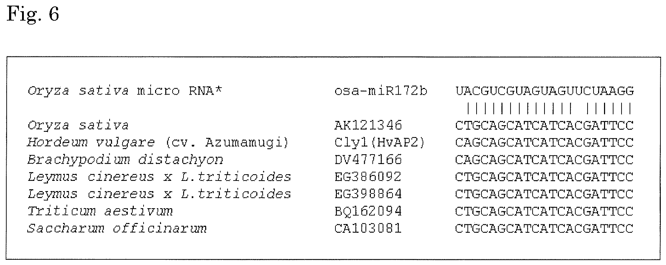

A comparison was made among base sequences of AP2 genes of barley varieties. As a result, only one single-nucleotide polymorphism associated with chasmogamy/cleistogamy of barley was found among the microRNA (miR172) target sites of the AP2 genes (Fig. 3B). For this reason, it was conceivable that this single-nucleotide polymorphism caused the difference in affinity between miR172 and the mRNA of the AP2 gene. In addition, mRNAs of AP2 genes (Cly1) of chasmogamous types underwent cleavage at the target site of microRNA, whereas mRNAs of the AP2 genes (cly1) of cleistogamous types did not undergo cleavage (Figs. 5D and 5E). These facts revealed that the presence or absence of cleavage of the mRNA of the AP2 gene determines the deference in chasmogamous/cleistogamous phenotype of barley. The base sequence of the miR172 target site is highly conserved also in chasmogamous plants in addition to barley, and these chasmogamous plants have base sequences which undergo cleavage with miR172 (Fig. 6). Accordingly, the present inventors have found that, by analyzing the base sequences of AP2 genes of plants including barley, it is possible to determine an open-flower pollination character or a closed-flower pollination character of the plants. Moreover, the present inventors have found that, by use of a cleistogamous-type AP2 gene (cly1), it is possible to impart a cleistogamous trait to plants including barley.

Accordingly, the present invention relates to a gene controlling chasmogamy/cleistogamy of a plant, as well as determination of chasmogamy/cleistogamy of a plant and production of a cleistogamous plant, which make use of the gene. More specifically, the present invention provides the following:
A DNA which does not undergo cleavage with a microRNA comprising the base sequence of SEQ ID NO: 10, and which imparts cleistogamy to a plant, wherein
   the DNA is described in any one of the following (a) to (c):
   (a) a DNA comprising a coding region of the base sequence shown in SEQ ID NO: 1, 2, 4, or 5;
   (b) a DNA comprising a base sequence in which one or a plurality of bases are substituted, deleted, added, and/or inserted in a coding region of the base sequence shown in SEQ ID NO: 1, 2, 4, 5, 7, or 8, and has a microRNA target site comprising a base sequence in which "a" at position 8 is substituted with "g" in comparison with the base sequence shown in SEQ ID NO: 11 or in which both "a" s at positions 2 and 14 are substituted in comparison with the base sequence shown in SEQ ID NO: 11; and
   (c) a DNA which hybridizes with a DNA comprising the base sequence shown in SEQ ID NO: 1, 2, 4, 5, 7, or 8 under stringent conditions, and has a microRNA target site comprising a base sequence in which "a" at position 8 is substituted in comparison with the base sequence shown in SEQ ID NO: 11 or in which both "a" s at positions 2 and 14 are substituted with "g" in comparison with the base sequence shown in SEQ ID NO: 11.
According to a preferred embodiment in the DNA according to the invention the substitution of the bases in the microRNA target site does not involve change in any encoded amino acid.

Further the invention refers to a vector comprising the DNA according to the invention.

Subject matter of the invention is also a plant cell into which the DNA according to the invention is introduced as an exogenous DNA and which has the DNA in homozygous manner. According to further aspects, the preset invention is directed to a plant which comprises the aforementioned plant cell and a plant which is a progeny or a clone of the aforementioned plant. The invention is also directed to a propagation material of the plant and to the method for producing a plant having a cleistogamous trait comprising a step of producing a plant which has the DNA according to the invention in a homozygous manner, by introducing the DNA as an exogenous DNA into the plant. Further the invention refers to a method for determining chasmogamy/cleistogamy of a plant comprising:
comparing a base sequence of a DNA of a test plant with a reference base sequence, wherein
   the DNA is described in any one of the following (a) to (c):
   (a) a DNA comprising a coding region of the base sequence shown in SEQ ID NO: 1, 2, 4, 5, 7, or 8;
   (b) a DNA comprising a base sequence in which one or a plurality of bases are substituted, deleted, added, and/or inserted in a coding region of the base sequence shown in SEQ ID NO: 1, 2, 4, 5, 7, or 8, and has a microRNA target site comprising a base sequence in which "a" at position 8 is substituted in comparison with the base sequence shown in SEQ ID NO: 11 or in which both "a" s at positions 2 and 14 are substituted in comparison with the base sequence shown in SEQ ID NO: 11; and
   (c) a DNA which hybridizes with a DNA comprising the base sequence shown in SEQ ID NO: 1, 2, 4, 5, 7, or 8 under stringent conditions, and has a microRNA target site comprising a base sequence in which "a" at position 8 is substituted in comparison with the base sequence shown in SEQ ID NO: 11 or in which both "a" s at positions 2 and 14 are substituted in comparison with the base sequence shown in SEQ ID NO: 11.

Finally the invention refers to a method for determining chasmogamy/cleistogamy of a plant, comprising detecting cleavage, with a microRNA comprising the base sequence shown in SEQ ID NO: 10, of a transcription product of the DNA according to the invention of a test plant.

### [Advantageous Effects of Invention]

According to the present invention, a novel gene Cly1/cly1 controlling chasmogamy/cleistogamy of a plant was identified, and the position of the gene on the chromosome, the structure of the gene, and the mechanism of chasmogamy/cleistogamy development were elucidated. As a result, provided are a method for determining chasmogamy/cleistogamy of a plant, targeting the Cly1/cly1 gene, and a method for breeding a cleistogamous plant, using the determination method. Moreover, a method for producing a cleistogamous plant variety, using the cly1 gene, is provided. The Cly1/cly1 gene controlling chasmogamy/cleistogamy is focused in the determination of chasmogamy/cleistogamy of a plant and the production and the breeding of a cleistogamous plant variety according to the present invention. Hence, these are more accurate than conventional methods using markers linked with the gene. For this reason, these methods make it possible to determine chasmogamy/cleistogamy of a plant, to produce or breed a cleistogamous plant variety more specifically and more efficiently than conventional methods.

### [Brief Description of Drawings]

[Fig. 1] Fig. 1 shows comparative maps of Cly1/cly1 genes.
[Fig. 2] Fig. 2 shows high resolution maps of the Cly1/cly1 genes.
[Fig. 3] Fig. 3 shows positional cloning of the Cly1/cly1 genes, where A is a genetic map constructed from an Azumamugi (AZ) × Kanto Nakate Gold (KNG) F2 population, and B shows sequence comparisons between alleles of the miR712 target site.
[Fig. 4] Fig. 4 shows phylogeny of AP2 homologs from A. thaliana, rice, maize, wheat, and barley.
[Fig. 5] Fig. 5 shows expression of the Cly1/cly1 gene, where A shows the 3' end of the gene, including Exon 10 which has the miR172 target site, B is a photograph showing gene expression in an immature spike at the awn primordium stage of Azumamugi (AZ), C is a photograph of electrophoresis showing gene expression in an immature spike throughout a series of developmental stages, D is a photograph of electrophoresis showing results of detection of transcription products cleaved by modified 5'RACE, and E shows a cleavage site of miR172.
[Fig. 6] Fig. 6 is a diagram showing miR172 target sites of Cly1/cly1 homologs of grass species.
[Fig. 7] Fig. 7 is a diagram showing a phylogenetic analysis of the Cly1/cly1 gene sequence of barley.
[Fig. 8] Fig. 8 is a diagram showing polymorphic sites in the Cly1/cly1 gene sequence of barley.

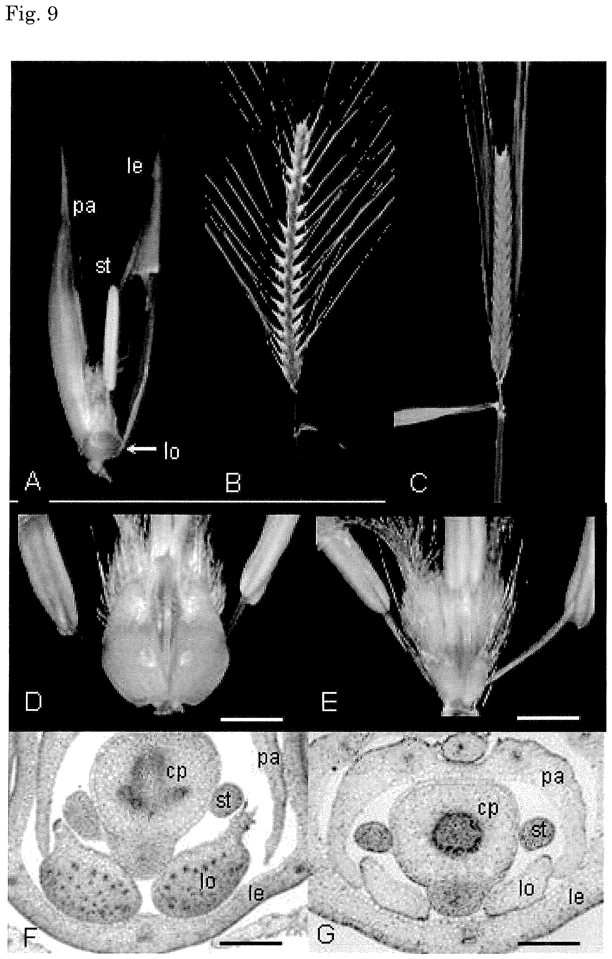

[Fig. 9] Fig. 9 shows photographs showing lodicules of chasmogamous barley and cleistogamous barley, where A is a photograph showing a morphology in which a lodicule (lo) located at the base of a stamen (st) opens a spikelet by pushing apart a lemma (le) and a palea (pa), B shows a spike of chasmogamous barley at anthesis, C shows a spike of cleistogamous barley at anthesis, D shows a lodicule of chasmogamous barley, E shows a lodicule of cleistogamous barley, F shows a section of a spikelet of chasmogamous barley, G shows a section of a spikelet of cleistogamous barley, and carpels (cp) are surrounded by other flower organs.

### [Description of Embodiments]

The present invention provides a DNA (hereinafter referred to as "cleistogamous-type DNA") encoding which imparts a cleistogamous trait to a plant. A cly1 DNA of the present invention does not undergo cleavage with miR172 (a microRNA comprising the base sequence shown in SEQ ID NO: 10), and, when expressed in a plant, imparts a cleistogamous trait to the plant.

A base sequence of the cly1 cDNA derived from a cleistogamous barley variety "Kanto Nakate Gold" is shown in SEQ ID NO: 1, a base sequence of a cly1 genomic DNA derived therefrom is shown in SEQ ID NO: 2, and an amino acid sequence of a protein encoded by these DNAs is shown in SEQ ID NO: 3. These sequences were identified by the present inventors. In addition, a base sequence of a cly1 cDNA derived from a cleistogamous barley variety "SV230" is shown in SEQ ID NO: 4, a base sequence of a cly1 genomic DNA derived therefrom is shown in SEQ ID NO: 5, and an amino acid sequence of a protein encoded by these DNAs is shown in SEQ ID NO: 6. These sequences were also identified by the present inventors. Meanwhile, a base sequence of a Cly1 cDNA derived from a chasmogamous barley variety "Azumamugi" is shown in SEQ ID NO: 7, a base sequence of a Cly1 genomic DNA derived therefrom is shown in SEQ ID NO: 8, and an amino acid sequence of a protein encoded by these DNAs is shown in SEQ ID NO: 9. These sequences were also identified by the present inventors.

An embodiment of the cleistogamous-type DNA of the present invention is a DNA comprising a coding region of the base sequence shown in SEQ ID NO: 1 or 2). Another embodiment of the cleistogamous-type DNA of the present invention is a DNA comprising a coding region of the base sequence shown in SEQ ID NO: 4 or 5). Moreover, the cleistogamous-type DNA of the present invention encompasses DNAs (various DNAs based on degeneracy of codons) encoding a protein comprising the amino acid sequence shown in SEQ ID NO: 3 and DNAs (various DNAs based on degeneracy of codons) encoding a protein comprising the amino acid sequence shown in SEQ ID NO: 6, as long as these DNAs encode transcription products which do not undergo cleavage with miR172, and impart a cleistogamous trait to a plant.

As shown in Examples, a comparison between the base sequence of the cly1 gene derived from the chasmogamous barley variety "Azumamugi" and the base sequence of the cly1 gene derived from the cleistogamous barley variety "Kanto Nakate Gold" shows that a base at a single position in the miR172 target site is different between them (Fig. 3B). In addition, a comparison between the base sequence of the Cly1 gene derived from the chasmogamous barley variety "Azumamugi" and the base sequence of the cly1 gene derived from the cleistogamous barley variety "SV230" shows that bases at two positions in the miR172 target site are different between them (Fig. 3B). Other barley varieties whose miR172 target sites have the same base sequence as that of "Azumamugi" exhibit a chasmogamous trait, whereas other barley varieties whose miR172 target sites have the same base sequence as that of "Kanto Nakate Gold" or "SV230" exhibit a cleistogamous trait. Hence, it is conceivable that the base variation in the miR172 target site influences the cleavage of the transcription product (mRNA) with miR172, and determines chasmogamy/cleistogamy of barley. Actually, the cleavage of the transcription product of the cly1 gene of "Kanto Nakate Gold" with miR172 is suppressed when compared with that of the transcription product of the Cly1 gene of "Azumamugi" (Fig. 5D and 5E).

Under the current state of the art, those skilled in the art can carry out a modification of a base sequence of a Cly1 DNA of a specific chasmogamous barley variety (for example, Azumamugi) so that the cleavage of the transcription product of the base sequence with miR172 can be suppressed. It is also possible to carry out various modifications of a base sequence of a cly1 DNA of a specific cleistogamous barley variety (for example, Kanto Nakate Gold, SV230), as long as the suppression of the cleavage of the transcription product of the base sequence with miR172 is maintained. In addition, mutation in the base sequence may occur even in the natural world. Accordingly, the present invention encompasses a DNA comprising a base sequence in which one or a plurality of bases are substituted, deleted, added, and/or inserted in a coding region of the base sequence (SEQ ID NO: 1, 2, 4, 5, 7, or 8) of the Cly1/cly1 gene of Azumamugi, Kanto Nakate Gold, or SV230, as long as the cleavage with miR172 is suppressed, and the DNA imparts a cleistogamous trait to a plant. Here, the term "a plurality" means generally 50 bases or less, preferably 30 bases or less, further preferably 10 bases or less, and several bases or less (for example, 5 bases or less, 3 bases or less, 2 bases or less, and 1 base) in the entire base sequence in the coding region of the gene.

The cleistogamous-type DNA of the present invention is preferably a DNA having a miR172 target site comprising abase sequence in which one or several bases are substituted in comparison with the base sequence shown in SEQ ID NO: 11 (the target sequence which undergoes cleavage with miR172). Here, the term "several bases" represents the number of bases in a range within which the cleavage with miR172 is suppressed, and is generally several bases or less (for example, 5 bases or less, 3 bases or less, 2 bases or less, or 1 base). The substitution of the bases in the miR172 target site is preferably one which does not involve change in any encoded amino acid. The substitution of the bases is, for example, substitution of at least one base selected from the group consisting of "a" at position 2, "a" at position 8, and "a" at position 14 in the base sequence shown in SEQ ID NO: 11. For example, the base sequence of the miR172 target sites in the cly1 genes of MG, GP, SV002, SV235, SV241, SV242, SV237, and SV235 is a base sequence in which "a" at position 8 is substituted with "g" in the base sequence shown in SEQ ID NO: 11 (Fig. 3B), as in the case with Kanto Nakate Gold. Meanwhile, for example, the base sequence of the miR172 target sites in the cly1 genes of SV223, SV253, and SV255 is a base sequence in which both "a"s at positions 2 and 14 are substituted with "c"s in the base sequence shown in SEQ ID NO: 11 (Fig. 3B), as in the case with SV230. The present invention encompasses the cly1 DNAs derived from these varieties.

Moreover, under the current state of the art, when a Cly1/cly1 gene is obtained from a specific barley variety (for example, Azumamugi, Kanto Nakate Gold, or SV230), those skilled in the art can obtain a DNA (hereinafter referred to as "homologous DNA") encoding a homologous gene controlling chasmogamy/cleistogamy from other plant by utilizing the base sequence information of the gene. Moreover, it is also possible to prepare a DNA which imparts a cleistogamous trait to a plant by modification of the base sequence of the obtained homologous DNA. Examples of the plant used to obtain such a homologous DNA include grass species such as barley, wheat, rye, Triticale, oat, rice, maize, and A. thaliana. The plant is preferably a plant of the family Poaceae such as barley, wheat, rye, Triticale, oat, rice, or maize, more preferably a plant of the subfamily Pooideae such as barley, wheat, rye, Triticale, oroat, and most preferably barley. In general, the obtained homologous DNA is capable of hybridizing with a DNA encoding a Cly1/cly1 gene of a specific barley variety (for example, Azumamugi, Kanto Nakate Gold, or SV230) under stringent conditions. Hence, the present invention also encompasses a DNA which hybridizes with a Cly1/cly1 DNA (SEQ ID NO: 1, 2, 4, 5, 7, or 8) of Azumamugi, Kanto Nakate Gold, or SV230 under stringent conditions, as long as the cleavage with miR172 is suppressed, and the DNA imparts a cleistogamous trait to a plant.

Whether or not a mutant DNA or a homologous DNA of the present invention undergoes cleavage with miR172 can be determined, for example, by an RNA ligase-mediated 5'RACE method described in Example (see Example 5(2)). Meanwhile, whether or not a mutant DNA or a homologous DNA imparts cleistogamy to a plant can be determined, for example, by recombining a cly1 gene of a chasmogamous variety with the DNA, producing a plant having the DNA in a homozygous manner, and checking whether or not a cleistogamous trait is imparted to the produced plant. The chasmogamy/cleistogamy of a plant can be determined by visually investigating whether or not an anther projects from a glumous flower at anthesis. Specifically, a plant can be determined to be chasmogamous, when an anther projects from a glumous flower at anthesis, whereas a plant can be determined to be cleistogamous, when an anther does not project from a glumous flower at anthesis. The chasmogamy/cleistogamy can also be determined by measuring a lodicule size at anthesis, because such deference in chasmogamy/cleistogamy is based on the difference in lodicule size. Specifically, a plant can be determined to be chasmogamous, when a lodicule swells and increases in size at anthesis, whereas a plant can be determined to be cleistogamous, when a lodicule remains small at anthesis (Fig. 9).

The cleistogamous-type DNA of the present invention is an agent for imparting a cleistogamous trait to a plant, in a sense that the introduction of the cleistogamous-type DNA makes it possible to impart a cleistogamous trait to a plant.

Note that the introduction of artificial mutation into a DNA for preparing the above-described mutant DNA can be carried out by, for example, the site-directed mutagenesis method (Kramer, W. & Fritz, HJ., Methods Enzymol, 1987, 154, 350).

Meanwhile, examples of a method for isolating the above-described homologous DNA include the hybridization technology (Southern, E. M., J. Mol. Biol., 98: 503, 1975) and the polymerase chain reaction (PCR) technology (Saiki, R. K., et al. Science, 230: 1350-1354, 1985, Saiki, R. K. et al. Science, 239: 487-491, 1988). In order to isolate a homologous DNA, a hybridization reaction is carried out under stringent conditions, in general. Examples of the stringent hybridization conditions include conditions of 6 M urea, 0.4% SDS, and 0.5 x SSC, and hybridization conditions with similar stringency. When more stringent conditions, for example, conditions of 6 M urea, 0.4% SDS, and 0.1xSSC, are employed, isolation of a DNA with a higher homology can be expected. The isolated DNA has a sequence identity of at least 50% or higher, further preferably 70% or higher, further preferably 90% or higher (for example, 95%, 96%, 97%, 98%, or 99% or higher) at the nucleic acid level or the amino acid sequence level. The sequence homology can be determined by use of a program (Altschul et al. J. Mol. Biol., 215: 403-410, 1990) of BLASTN (nucleic acid level) or BLASTX (amino acid level). These programs are based on the algorithm BLAST by Karlin and Altschul (Proc. Natl. Acad. Sci. USA, 87:2264-2268, 1990, Proc. Natl. Acad. Sci. USA, 90: 5873-5877, 1993). When a base sequence is analyzed by BLASTN, parameters are set to, for example, score=100, and wordlength=12. Meanwhile, when an amino acid sequence is analyzed by BLASTX, parameters are, for example, set to score=50, and wordlength=3. When an amino acid sequence is to be analyzed by use of the Gapped BLAST program, the analysis can be conducted as described in Altschul et al., (Nucleic Acids Res. 25: 3389-3402, 1997). When BLAST and Gapped BLAST programs are used, the default parameters of the programs are used. The specific procedures of these analysis methods are known.

The form of the cleistogamous-type DNA of the present invention is not particularly limited, and the cleistogamous-type DNA encompasses genomic DNAs and chemically synthesized DNAs in addition to cDNAs. These DNAs can be prepared by routine methods for those skilled in the art. A genomic DNA can be prepared, for example, by extracting genomic DNAs from a plant, constructing a genomic library (a plasmid, a phage, a cosmid, a BAC, a PAC or the like can be used as the vector), spreading the genomic library, and conducting colony hybridization or plaque hybridization by use of a probe prepared based on a base sequence of a Cly1/cly1 gene (for example, the DNA shown in any one of SEQ ID NOs: 1, 2, 4, 5, 7, and 8). Alternatively, a genomic DNA can also be prepared by preparing a primer specific to a Cly1/cly1 gene, and conducting PCR using the primer. Meanwhile, a cDNA can be prepared, for example, by synthesizing cDNAs based on mRNAs extracted from a plant, inserting the cDNAs into a vector such as λZAP to prepare a cDNA library, spreading the cDNA library, and conducting colony hybridization or plaque hybridization in the similar manner as described above, or conducting PCR. In addition, if a commercially available DNA synthesizer is used, a desired DNA can be prepared by synthesis.

### <Vector, transformed sorghum cell, transgenic sorghum plant>

The present invention also provides a vector comprising the above-described cleistogamous-type DNA of the present invention, a plant cell into which the above-described cleistogamous-type DNA of the present invention or a vector comprising the cleistogamous-type DNA of the present invention is introduced, a plant comprising the plant cell, a plant which is a progeny or a clone of the plant, and propagation materials of these plants.

The vector of the present invention is not particularly limited, as long as the vector is capable of expressing the inserted gene in a plant cell. The vector of the present invention may comprise a promoter for constitutive or inducible expression of the DNA of the present invention. Examples of the promoter for constitutive expression include the 35S promoter of cauliflower mosaic virus, the actin promoter of rice, the ubiquitin promoter of maize, and the like. Meanwhile, examples the promoter for inducible expression include promoters which are known to initiate expression in response to external factors such as infection with or invasion of filamentous fungi·bacteria·viruses, low-temperature, high-temperature, dryness, ultraviolet irradiation, and spraying of particular compounds. Examples of these promoters include the promoter of the rice chitinase gene and the promoter of the tobacco PR protein gene, which are expressed by infection with or invasion of filamentous fungi·bacteria·viruses, the promoter of the rice lip19 gene, which is induced by low-temperature, the promoters of the rice hsp80 gene and hsp72 gene which are induced by high-temperature, the promoter of the A. thaliana rab16 gene, which is induced by dryness, the promoter of the parsley chalcone synthase gene, which is induced by ultraviolet irradiation, the promoter of the maize alcohol dehydrogenase gene, which is induced under anaerobic conditions, and the like. In addition, the promoter of the rice chitinase gene and the promoter of the tobacco PR protein gene are also induced by specific compounds such as salicylic acid, and the rab16 is also induced by spreading of a plant hormone, abscisic acid.

The present invention also provides a plant cell into which the vector of the present invention is introduced. The plant from which the plant cell is derived is not particularly limited, and examples thereof include grass species such as barley, wheat, rye, Triticale, oat, rice, maize, and A. thaliana. Plants of the family Poaceae such as barley, wheat, rye, Triticale, oat, rice, and maize are preferable, plants of the subfamily Pooideae such as barley, wheat, rye, Triticale, and oat are more preferable, and barley is most preferable. The plant cell of the present invention also encompasses cells in plants, in addition to cultured cells. In addition, the plant cell of the present invention encompasses plant cells in various forms, such as suspended cultured cells, protoplasts, leaf slices, calluses, immature embryos, and pollen. For introducing the vector into a plant cell, it is possible to use various methods known to those skilled in the art such as the polyethylene glycol method, electroporation, the Agrobacterium mediated method, and the particle gun method.

Regeneration of plants from transformed plant cells can be carried out by a method known to those skilled in the art which depends on the kind of the plant cells. For example, examples of methods for producing a transgenic barley plant include the methods described in Tingay et al., (TingayS. et al. Plant J. 11: 1369-1376, 1997), Murray et al., (Murray F et al. Plant Cell Report 22: 397-402, 2004), and Travalla et al., (Travalla S et al. Plant Cell Report 23: 780-789, 2005). Meanwhile, the following are several technologies which are already established as methods for producing a transgenic rice plant, and which have been widely used in the technical field of the invention of the present application: a method in which a plant is regenerated by introducing a gene into a protoplast with polyethylene glycol (Datta, S. K. In Gene Transfer To Plants (Potrykus I and Spangenberg Eds.) pp. 66-74, 1995), a method in which a plant is regenerated by introducing a gene into a protoplast with an electric pulse (Toki et al. Plant Physiol. 100, 1503-1507, 1992), a method in which a plant is regenerated by directly introducing a gene into a cell by the particle gun method (Christou et al. Bio/technology, 9: 957-962, 1991), and a method in which a plant is regenerated by introducing a gene with Agrobacterium (Hiei et al. Plant J. 6: 271-282, 1994), and the like. Meanwhile, an example of a method for producing a transgenic A. thaliana plant is the method of Akama et al., (Akama et al. Plant Cell Reports 12: 7-11, 1992). In the present invention, these methods can be used suitably.

Once a transgenic plant whose genome has the cleistogamous-type DNA of the present invention introduced therein is obtained, a progeny can be obtained from the plant by sexual reproduction or asexual reproduction. Moreover, it is also possible to obtain a propagation material (for example, seeds, fruits, cut-spikes, stubbles, calluses, protoplasts, or the like) from the plant or a progeny or clone thereof, and then to mass produce the plant from the propagation material. The present invention encompasses plant cells into which the DNA of the present invention is introduced, plants comprising the cells, progenies and clones of the plants, propagation materials of the plants and progenies and clones thereof.

### <Method for producing plant to which cleistogamous trait is imparted>

The present invention also provides a method for producing a plant to which a cleistogamous trait is imparted, comprising introducing the cleistogamous-type DNA of the present invention into a plant. The cleistogamous-type DNA of the present invention can be introduced into a plant as an exogenous DNA. In addition, in the present invention, "to impart" a cleistogamous trait to a plant has a meaning including not only to give a cleistogamy to a variety having a chasmogamous trait, but also to further enhance cleistogamy of a variety already having certain cleistogamy. In order to impart a cleistogamous trait to a plant, both cly1 alleles in an individual are preferably converted into cleistogamous-type DNAs. The introduction of the cleistogamous-type DNA of the present invention into a plant genome can be carried out by, for example, mating or homologous recombination. A plant to which a cleistogamous trait is imparted is, for example, excellent in ability to prevent infection with Fusarium ear blight, or excellent in ability to suppress pollen dispersal, in comparison with plants having no such a trait.

### <Method for determining chasmogamy/cleistogamy of plant>

### -Analysis of base sequence of Cly1/cly1 gene-

The present invention also provides a method for determining chasmogamy/cleistogamy of a plant. An embodiment of the determination method of the present invention is a method comprising: analyzing a base sequence of a Cly1/cly1 gene in a plant; and comparing the analyzed base sequence with a reference base sequence. The Cly1/cly1 gene in the plant is typically a gene comprising any one of the following DNAs, because it is conceivable that the Cly1/cly1 gene in the plant is generally homologous to a Cly1/cly1 gene of a specific barley variety (for example, Azumamugi, Kanto Nakate Gold, or SV230):
(a) a DNA comprising a coding region of the base sequence shown in SEQ ID NO: 1, 2, 4, 5, 7, or 8;
(b) a DNA comprising a base sequence in which one or a plurality of bases are substituted, deleted, added, and/or inserted in a coding region of the base sequence shown in SEQ ID NO: 1, 2, 4, 5, 7, or 8; and
(c) a DNA which hybridizes with a DNA comprising the base sequence shown in SEQ ID NO: 1, 2, 4, 5, 7, or 8 under stringent conditions

Here, the definitions of the terms "a plurality of" and "stringent conditions" are as described above. The meaning of the "analyzing a base sequence of a gene" includes not only analysis of the entire length of the base sequence of the gene, but also analysis of the base sequence of a specific part of the gene. The base sequence of the specific part preferably comprises a base sequence of a miR172 target site of the gene.

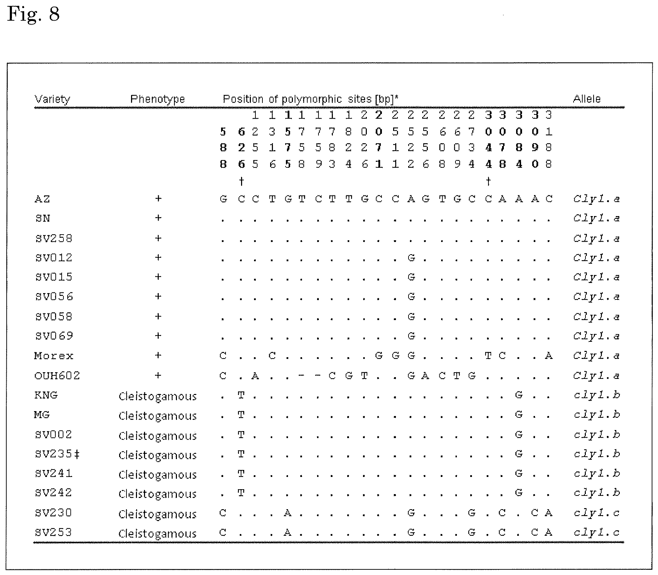

For analysis of the base sequence of the Cly1/cly1 gene, an amplification product obtained by amplifying a DNA of the Cly1/cly1 gene by PCR can be used. In the case where PCR is carried out, a primer used is not particularly limited, as long as the primer is capable of specifically amplifying the Cly1/cly1 gene. The primer can be designed as appropriate on the basis of the sequence information of the Cly1/cly1 gene (for example, the base sequence shown in SEQ ID NO: 1, 2, 4, 5, 7, or 8, a base sequences shown in Fig. 8). It is possible to use not only a primer amplifying the entire length of the DNA of the Cly1/cly1 gene, but also a primer amplifying a DNA of a specific part. The primer is preferably one at least capable of amplifying a DNA comprising a base sequence of a miR172 target site.

The "reference base sequence" to be compared with the base sequence of the Cly1/cly1 gene in the test plant is typically a base sequence of a Cly1/cly1 gene for which whether the Cly1/cly1 gene is of a chasmogamous-type or of a cleistogamous-type has already been determined. The "reference base sequence" may be the base sequence of the Cly1/cly1 gene (for example, the base sequence shown in SEQ ID NO: 1, 2, 4, 5, 7, or 8, the base sequence shown in Fig. 8) already identified by the present inventors. Preferably, the "reference base sequence" is a base sequence of a miR172 target site of a Cly1/cly1 gene (for example, the base sequence shown in Fig. 6).

A comparison between a determined base sequence of the gene in the test plant and the reference base sequence enables a judgment as to whether the gene in the test plant is of a chasmogamous-type or of a cleistogamous-type. For example, a probability that the gene of the test plant is of a chasmogamous-type is determined to be high, when the base sequence of the gene is substantially identical to a base sequence of a Cly1 gene of a chasmogamous-type variety (for example, the base sequence shown in SEQ ID NO: 7 or 8, the Cly1a base sequence shown in Fig. 8) (particularly when a base sequence of a miR172 target site is identical, or when the difference in a base sequence of a miR172 target site is so small that it is conceivable that cleavage of a transcription product with miR172 is not suppressed). On the other hand, the probability that the gene in the test plant is of a cleistogamous-type is determined to be high, when the base sequence of the gene is substantially identical to a base sequence of a cly1 gene of a cleistogamous-typevariety (for example, the base sequence shown in SEQ ID NO: 1, 2, 4, or 5, the cly1b or cly1c base sequence shown in Fig. 8) (particularly when a base sequence of a miR172 target site is identical or when the difference in a base sequence of a miR172 target site is so small that is conceivable that cleavage of a transcription product with miR172 is suppressed).

Whether or not the base sequence of the gene in the test plant is different from the reference base sequence can be indirectly analyzed by various methods, besides the above-described direct determination of the base sequence. Examples of these methods include the PCR-SSCP (single-strand conformation polymorphism) method, the RFLP method and the PCR-RFLP method which make use of restriction fragment length polymorphism (RFLP), denaturant gradient gel electrophoresis (DGGE), the allele specific oligonucleotide (ASO) hybridization method, and the ribonuclease A mismatch cleavage method.

Note that, in the determination method of the present invention, the preparation of the DNA from the test plant can be conducted by an ordinary method, for example, by the CTAB method. The source from which the DNA is obtained is not particularly limited, and the DNA can be extracted from any tissue of the plant. For example, the DNA can be extracted from spikes, leaves, roots, stems, seeds, albumen portions, embryos, or the like. For the preparation of the DNA, not only grown plants, but also plant seeds and seedlings can be used. Hence, it is possible to determine chasmogamy/cleistogamy of a plant at an early stage.

In addition, the base sequencing can be carried out by an ordinary method, for example, the dideoxy method, the Maxam-Gilbert method, or the like. For the base sequencing, a commercially available sequencing kit and sequencer can be used.

### -Detection of cleavage of transcription product of gene with miR172-

Another embodiment of the determination method of the present invention is a method comprising detecting cleavage, with miR172, of a transcription product of a gene of a test plant. The detection of the cleavage, with miR172, of a transcription product of a gene can be carried out by detecting a transcription product shortened by the cleavage. For example, the RNA ligase-mediated 5'RACE method described in Example can be used for the detection of the shortened transcription product. Specifically, an RNA oligomer which binds to only the 5'end of a cleaved transcription product (a fragment on the 3' side) is caused to act on total RNA extracted from a plant, and then a nested PCR is performed by use of a primer for the RNA oligomer and a reverse primer specific to the cleaved transcription product (the fragment on the 3' side). Thus, the transcription product cleaved with miR172 can be detected specifically. A commercially available kit (for example, GeneRacer Kit (Invitrogen)) can be used for this method.

It is conceivable that, once the transcription product of the gene is cleaved with miR172, a subsequent translation process is suppressed. Hence, the detection of cleavage, with miR172, of a transcription product of a gene can also be carried out by detecting a translation product of the gene, besides the above-described direct detection method of a transcription product. The detection of the translation product of the gene can be carried out by an ordinary method, for example, by the Western blotting method. An antibody used for the Western blotting may be a polyclonal antibody or a monoclonal antibody. Preparation methods of these antibodies are well-known to those skilled in the art. For example, a protein comprising the amino acid sequence shown in SEQ ID NO: 3, 6, or 9 or a partial peptide thereof can be used as an antigen used to prepare the antibody.

The probability that the test plant is chasmogamous is determined to be high, when cleavage, with miR172, of a transcription product of a gene is detected for the test sample, as a result of the above-described method. On the other hand, the probability that the test plant is cleistogamous is determined to be high, when no cleavage is detected.

### <Method for breeding cleistogamous plant>

Also described herein is a method for breeding a cleistogamous plant. The breeding method comprises the methods of:
(a) mating a chasmogamous plant variety with any plant variety;
(b) determining, by the above-described determination method of the present invention, chasmogamy/cleistogamy of individuals which are obtained by the mating; and
(c) selecting a variety which is determined to have cleistogamy.

Examples of the "any plant variety" to be mated with the chasmogamous plant variety include cleistogamous varieties, and individuals obtained by mating a cleistogamous variety with a chasmogamous variety, but the "any plant variety" is not limited to these. The use of the breeding method described herein makes it possible to select cleistogamous plants at the seed stage or the seedling stage. Hence, development of a variety having a cleistogamous trait can be performed in a short time. Moreover, it can be said that the breeding method is highly accurate, because the selection is carried out by not employing a linkage marker molecule as a target, but employing the gene controlling chasmogamy/cleistogamy of a plant as a direct target.

### [Examples]

Hereinafter, the present invention will be described more specifically on the basis of Examples. However, the present invention is not limited to Examples below.

### (Example 1) Construction of comparative genetic maps between barley and rice

One hundred and thirty seven barley EST sequences were matched with sequences on chromosome 4 of rice. As a result, 24 sequences were able to be mapped to chromosome 2H of barley. Of the 24 sequences, 18 sequences were converted to STS markers, 4 sequences were converted to SNP markers, and the remaining two sequences were converted to SSR markers (Table 1).

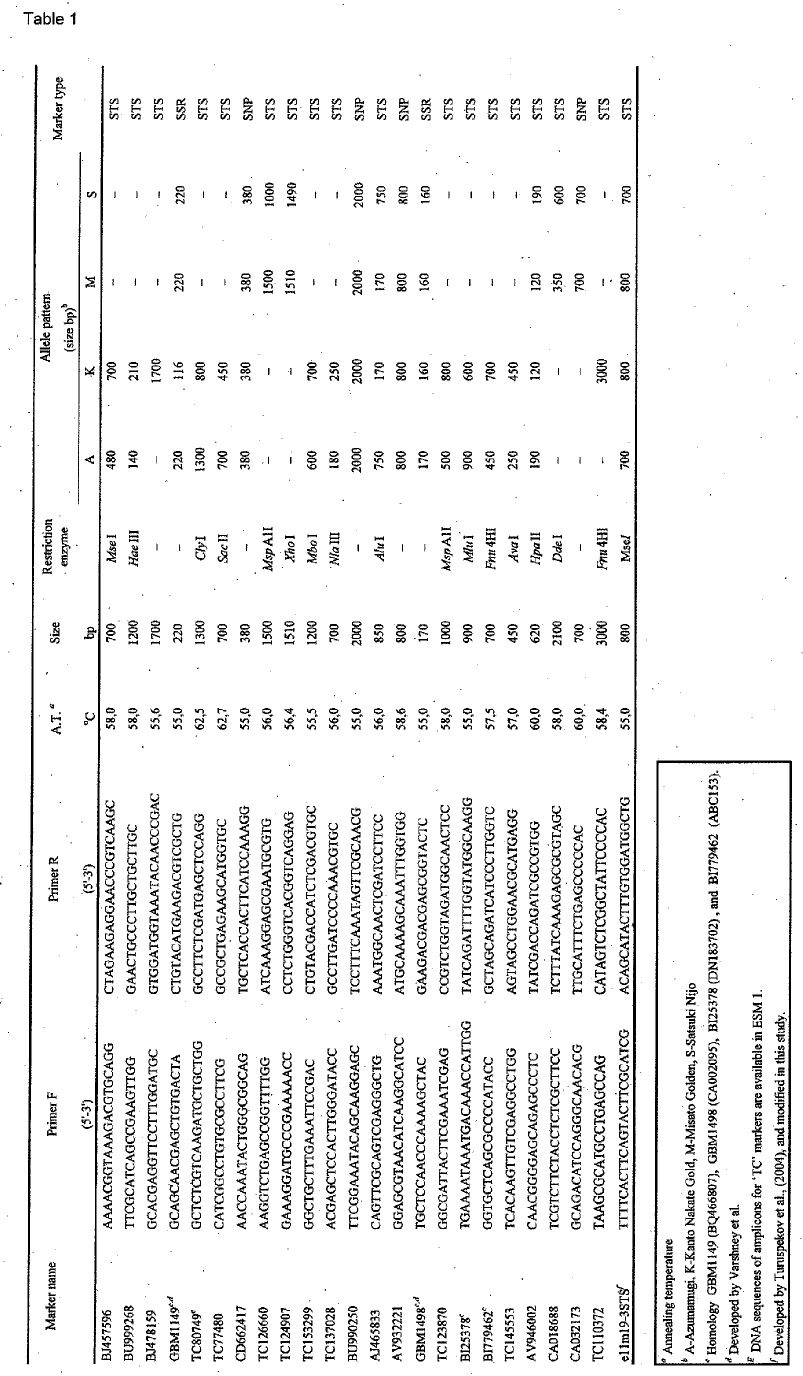

Fig. 1 shows a comparison between a physical map of chromosome 4 of rice and genetic maps of barley, which are created by the present inventors. Among Kanto Nakate Gold (KNG) x Azumamugi (AZ) individuals, cly1 co-segregated from MSU21, e11m19-3STS, AJ465833, and GBM1498. A BLAST search of these sequences against the genomic sequence of chromosome 4 of rice revealed that GBM1498 and AJ465833 are homologous to RM1153 and C1016, respectively. Meanwhile, neither MSU21 nor e11m19-3STS was related to any genomic sequence of rice. Sequences between 30.13 Mb and 34.68 Mb of chromosome 4 of rice were co-linearly arranged in barley, whereas the region from 31.66 Mb to 33.52 Mb was inverted in barley. Several other disruptions in gene order were detected.

### (Example 2) Genotypic analysis of F2 population

By a genetic analysis of a large-scale KNG × AZ F2 population (2652 individuals), the cly1 gene locus was mapped to a position 0.66 cM distal from AV932221 and 0.15 cMproximal from CA002095 (Fig. 3A). AV932221 andCA002095 were homologous to Os04g0650000 and Os04g0648900 of rice, respectively, and 11 genes were found between these two loci (http://rapdb.dna.affrc.go.jp/). It was speculated, on the basis of a partial cDNA sequence, that one of these, Os04g0649100, encoded an AP2 protein. BF623536 was a barley EST homologous to Os04g0649100. On the assumption that BF623536 was a part of Cly1, the Morex BAC library (Yu Y, et al., Theor Appl Genet 101: 1093-1099, 2000) was PCR-screened with the sequence of BF623536 to identify the full genomic sequence of Cly1. As a result, five clones (M060H23, M191K21, M601E22, M799P16, and M796O24) were selected, and the base sequences of two (M191K21 and M601E22) of the five clones were determined. On the basis of the sequences of these two BAC clones, further five new markers were prepared (Table 2), and mapped (Fig. 3A).

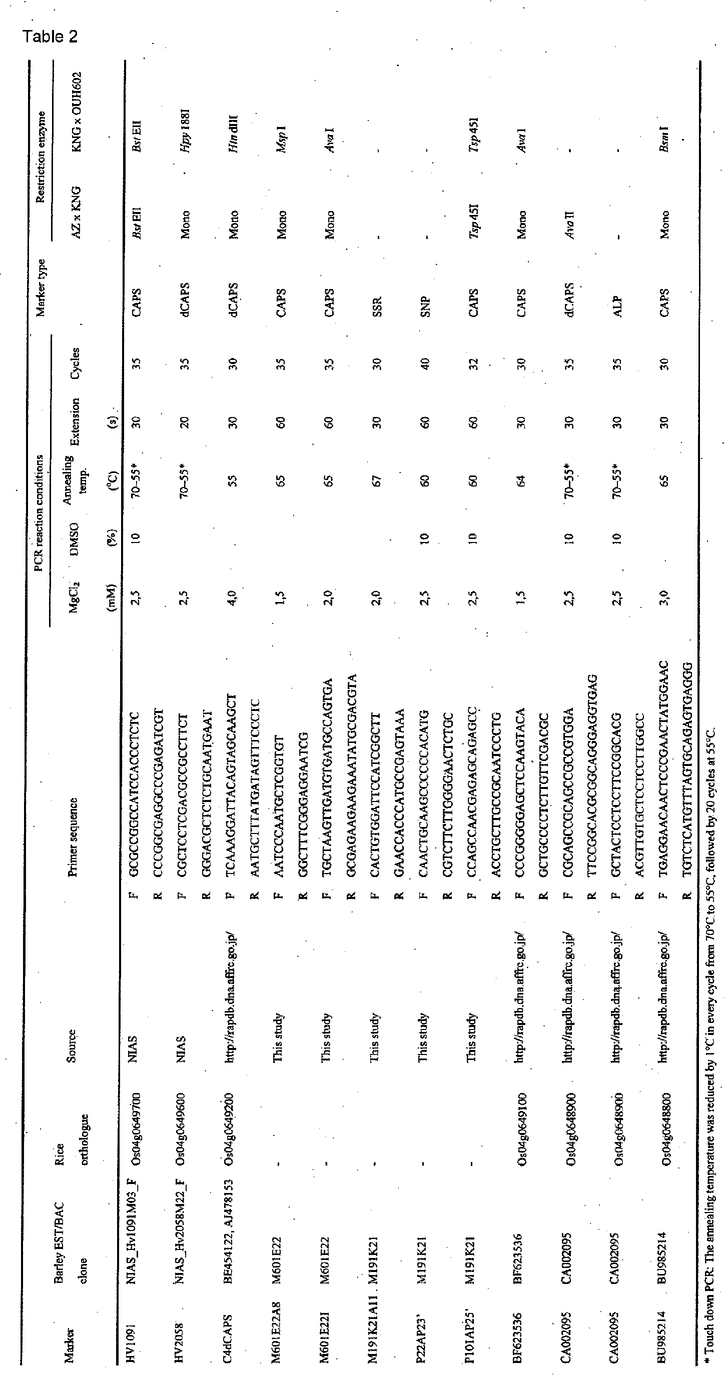

A single recombination event (0.02 cM) separated P101AP25' from cly1, and two recombination events (0.04 cM) separated M191K21A11 from cly1. These results narrowed the genomic region containing cly1 to 7 kbp. The DNA region included most of the coding region of Cly1, and no other genes were present in the DNA region. A sequence (Cly1.a) in AZ differed from a sequence (cly1.b) of KNG at two base positions.

### (Example 3) Analysis of gene base sequence

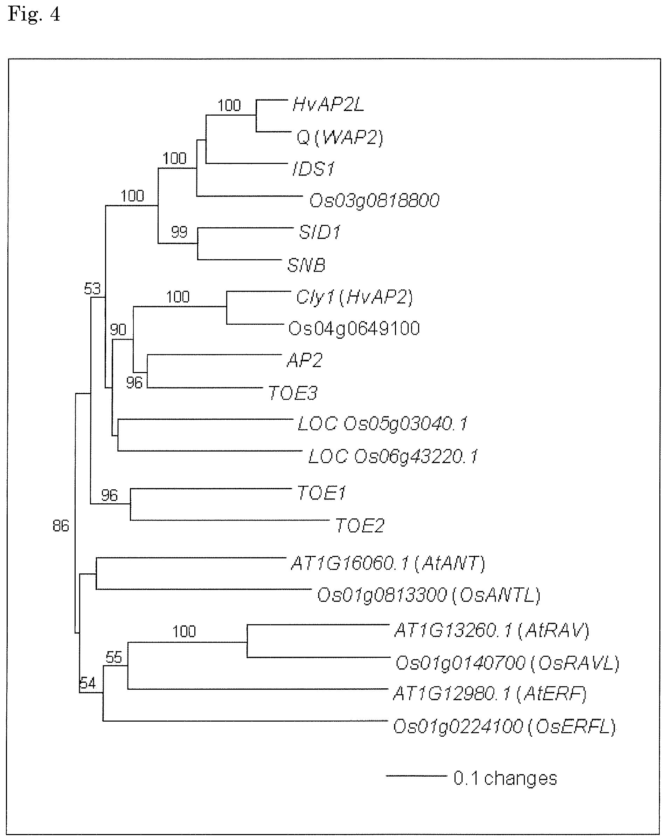

The Cly1 gene had 2691 bp from the start codon to the stop codon, had a GC content of 60.8%, and was divided into 10 exons and 9 introns (Fig. 3A). The coding sequence had 1464 bp, and flanked by a 5'UTR (480 bp) and a 3'UTR (346-368 bp). The Cly1 encoded a 487-residue polypeptide having two AP2 domains (Jofuku KD, et al., Plant Cell 6: 1211-1225, 1994, Okamuro JK, et al., Proc Natl Acad Sci USA 94: 7076-7081, 1997). One of the AP2 domains was present between positions 112 and 178, and the other one was present between positions 203 and 270. A putative miR172 target sequence was present in Exon 10 (Fig. 3A), and the sequence is also present in many AP2 genes (Aukerman MJ, Sakai H 15: 2730-2741, 2003, Chen X Science 303: 2022-2025,2004). Two auxin-response elements (Guilfoyle T. et al. , How does auxin turn on genes? Plant Physiol 118: 341-347, 1998) were present upstream of the start codon (one was present within 3 k bp upstream, and the other was present within2 k bp upstream). The base sequence of the Cly1 gene resembled those of Arabidopsis. thaliana (A. thaliana) AP2 (AT4G36920.1) and TOE3 (AT5G67180.1), and belonged to the euAP2 clade (Fig. 4). The transcription product was highly homologous to the rice AP2-like gene Os04g0649100. The single-nucleotide polymorphism at P22AP23' (present in the putative miR172 target sequence within Exon 10) does not involve change in any amino acid.

### (Example 4) Polymorphism analysis of miR172 target site

The sequence polymorphism of the miR172 target site was associated with lodicule size and cleistogamy. Sequence variation among 274 barley lines was found at three base positions within the miR172 target sequence (Fig. 3B and Tables 3 to 7).

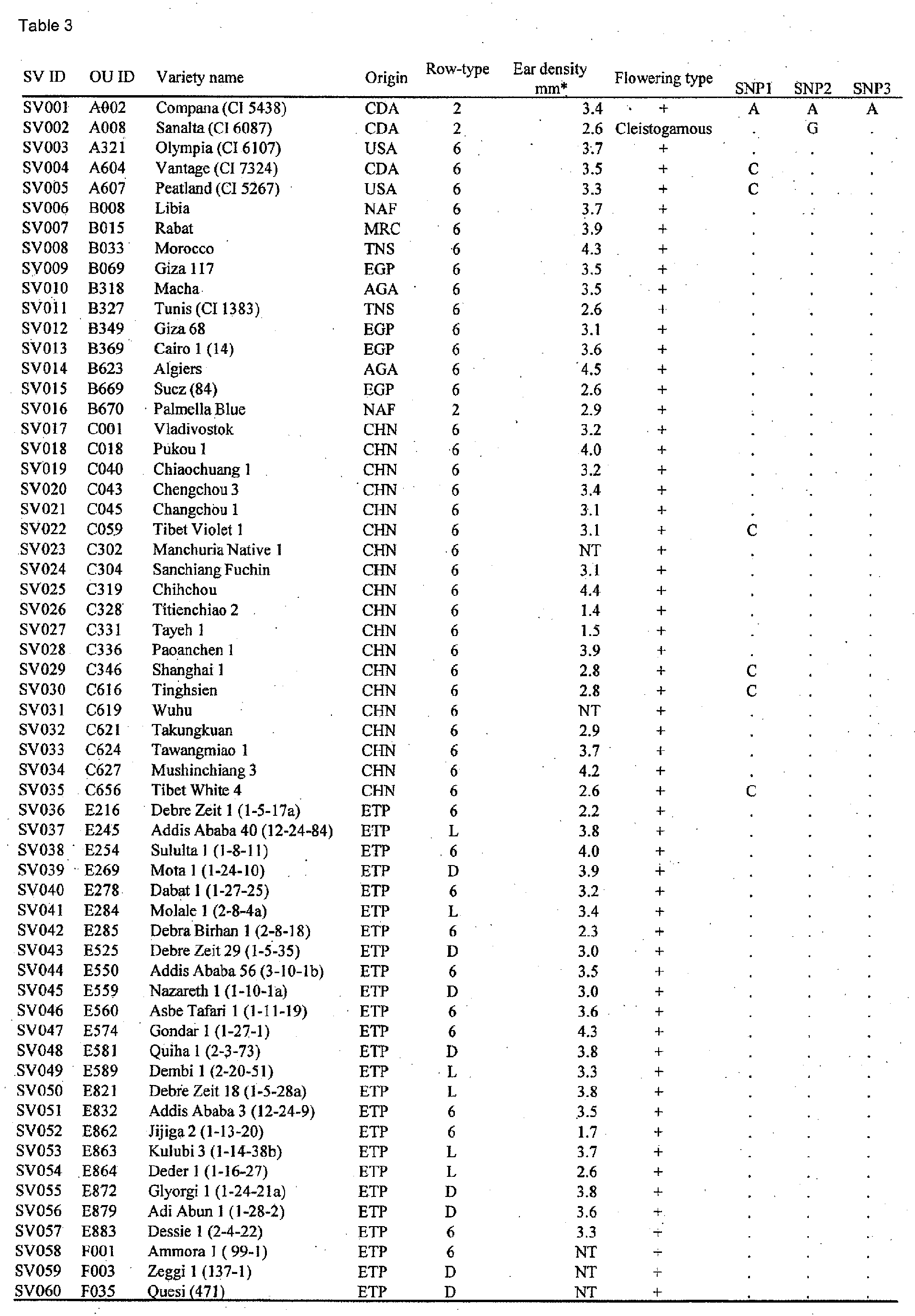

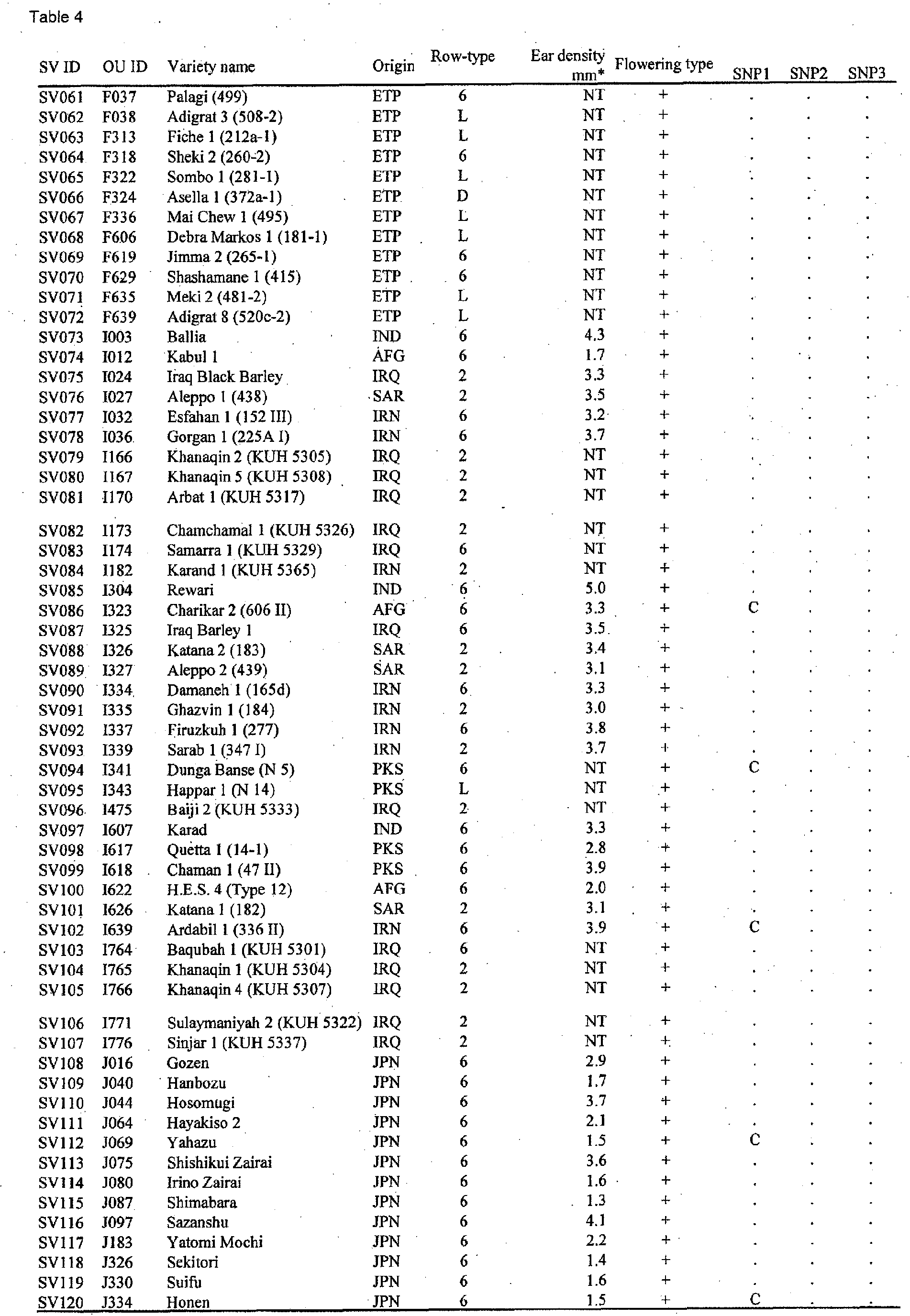

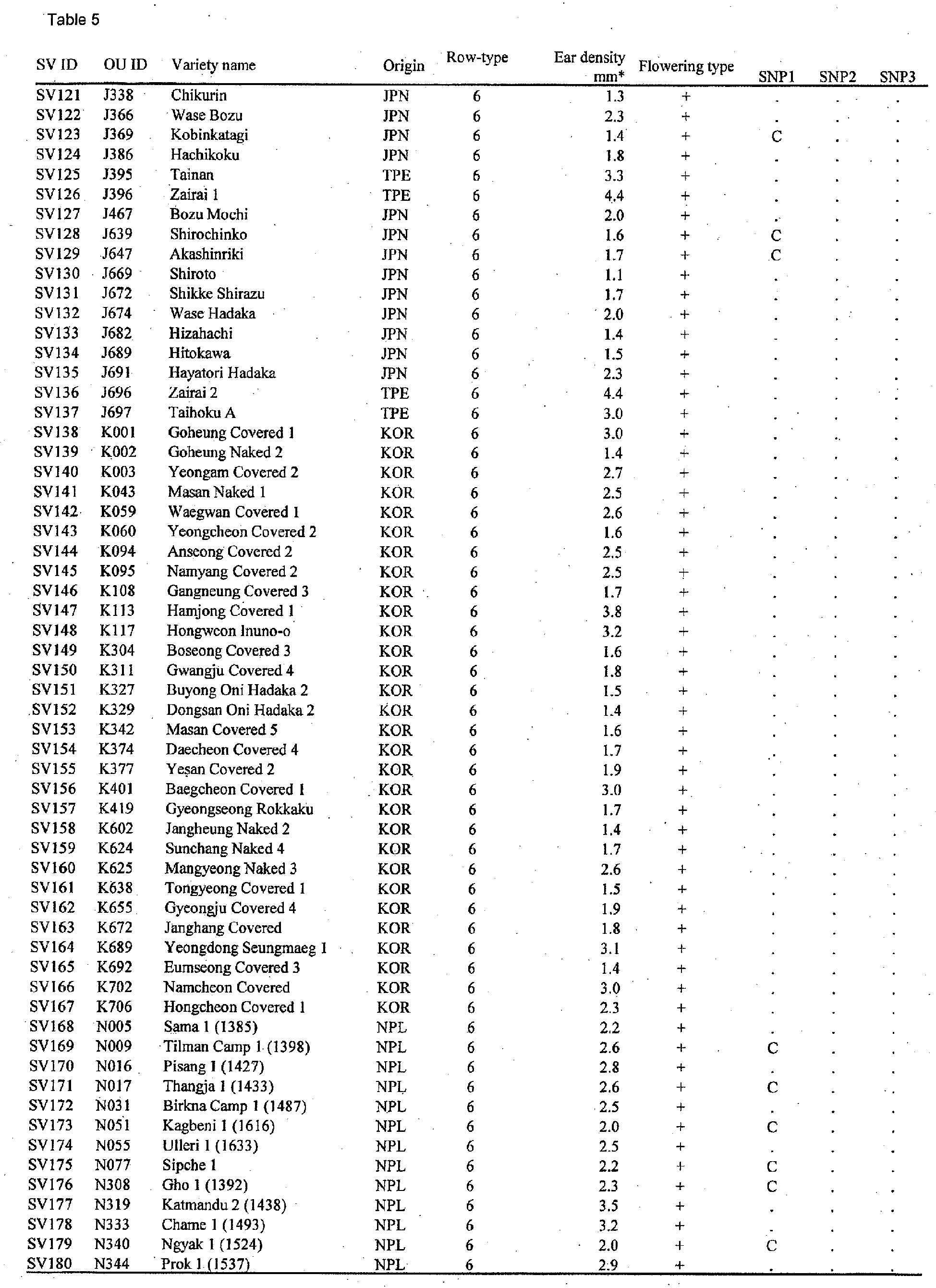

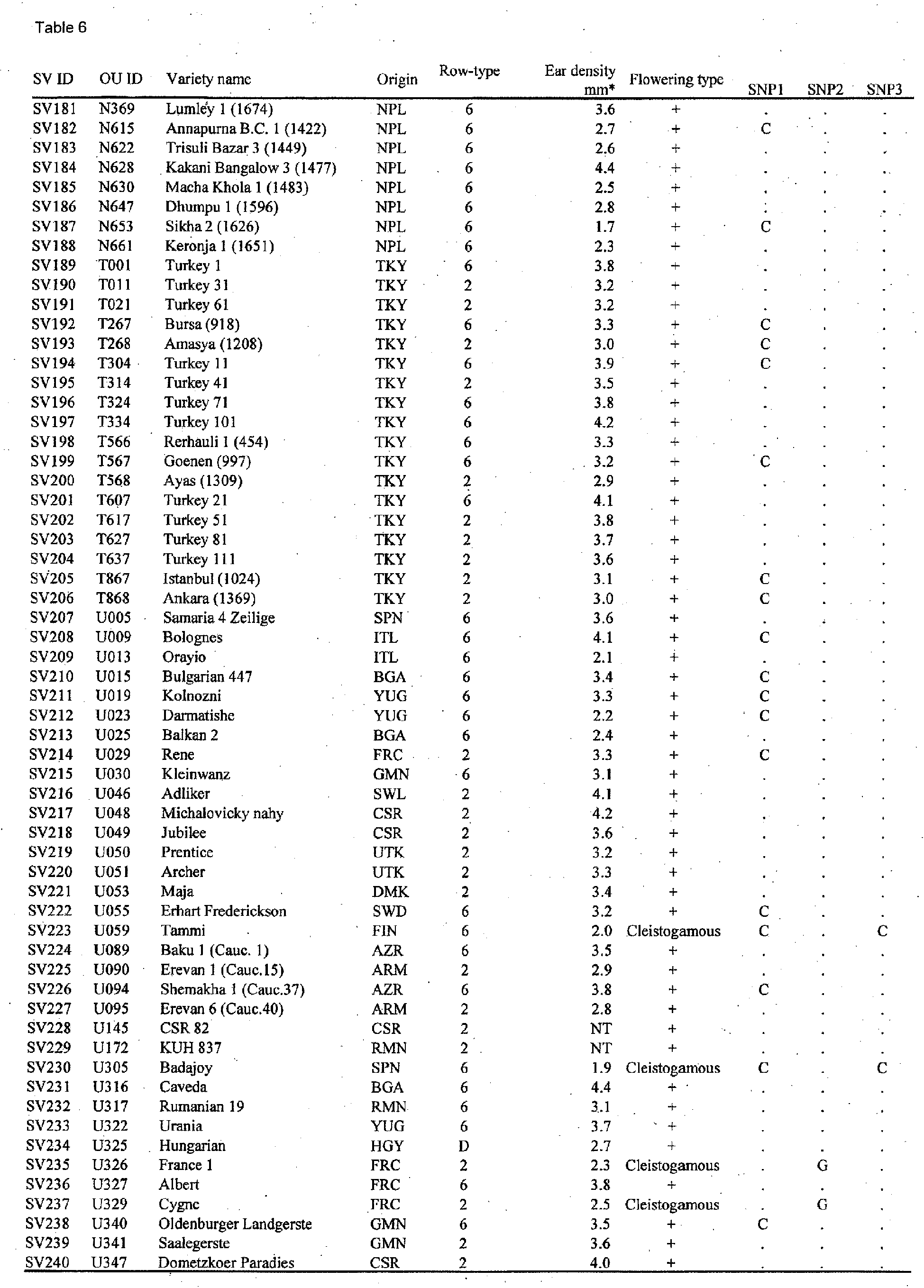

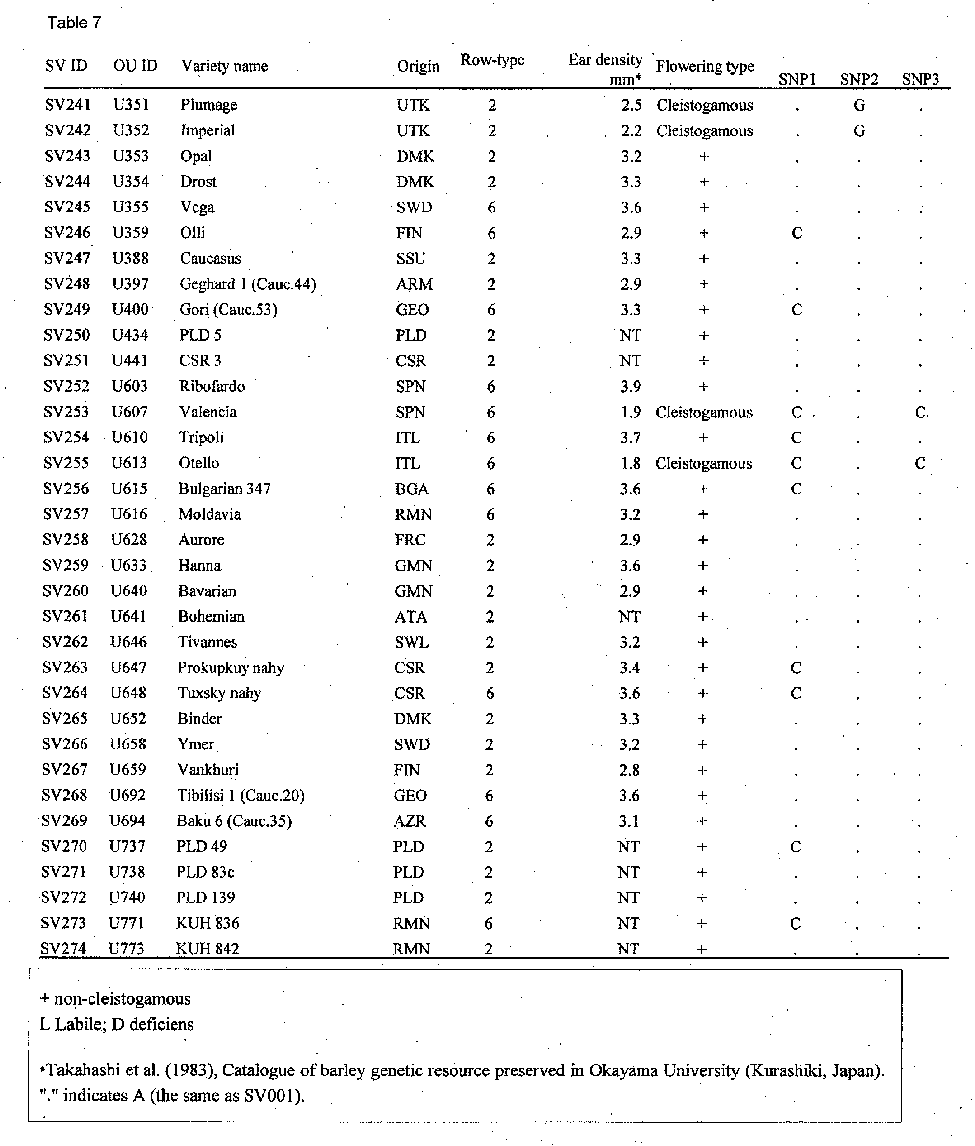

The sequence variation was present at the third base position of each codon, and hence did not bring about any variation in peptide sequence. A first variant "...C(A/C)G ..." was uncorrelated with cleistogamy, whereas second and third variants "... (A/G) TCATC (A/C)..." were correlated. The fact that all the noncleistogamous types were of haplotype "ATCATCA" at the latter site suggested that this haplotype was necessary for the determination of noncleistogamy (Fig. 3B). The haplotype of the cleistogamous types was either "GTCATCA" or "ATCATCC" (Fig. 3B). The initial G was present in seven cleistogamous populations in addition to KNG. Hence, it was suggested that this change in a single base was sufficient for the change from noncleistogamy to cleistogamy. A second cly1 allele had the alternative haplotype "ATCATCC" (where a C replaces an A in comparison with the noncleistogamous type). This haplotype was present in four cleistogamous populations (Fig. 3B).

### (Example 5) Analysis of cleavage of Cly1 mRNA with miR172

(1) RNAs were extracted from spikes at the awn primordium stage, and first strand cDNA synthesis was performed by use of SuperScipt II (Invitrogen). RT-PCR was conducted by use of a primer corresponding to one of the miR172 target sites (BF623536U514M060H23U540 "ACCAGCAGCAGCAACAGAGGC/SEQ ID NO: 12" and BF623536U514M060H23L919"GCTGGTAATGGCTGTGGGACG/ SEQ ID NO: 13") or one of the 3'UTRs (BF623536U514U794 "TCAAGAGCAGCCAGCCAAGAA/SEQ ID NO: 14" and BF623536U514M060H23L1053 "CCGCATCCGTCCTCCTCTCAA/SEQ ID NO: 15"). As a result, the gene expression pattern was essentially the same between an individual having the Cly1.a (AZ) allele and an individual having the cly1. b (KNG) allele (Fig. 5C). The expression level was slightly higher in KNG than AZ, but no significant change with time was identified by RT-PCR (Fig. 5C). The transcription level of gene fragment in the miR172 target site was similar to the transcription level of 3'UTR gene fragment.
(2) Modified 5'RACE was carried out to investigate the cleavage of Clyl mRNA with miR172. An RNA ligase-mediated 5'RACE (Kasschau KD, et al. Dev Cell 4: 205-217, 2003) using the GeneRacer Kit (Invitrogen) was applied to total RNA extracted from whole spikes at the awn primordium stage. Dephosphorylation and decapping steps were omitted, so that only the 5' ends of the cleaved transcription products were able to be ligated to the GeneRacer RNA oligomer. A nested PCR employed a primer targeting the GeneRacer RNAoligomer, initially in combination with a gene specific reverse primer (BF623536U514M060H23L1053), and subsequently in combination with a primer (BF623536U514M060H23L1031"TCCATCTCTCGCTCTCACCCA/SEQ ID NO: 16"). PCR products were separated by agarose gel electrophoresis, and cloned into the TA vector (Invitrogen). Randomly selected clones without any prior size selection were used for DNA base sequencing.

As a result, the nested PCR targeting the miR172 target site produced an amplicon with 400 bp or less from an individual having Cly1.a, but not from an individual having cly1.b (Fig. 5D). This suggests that the cleavage with miR172 occurred in the former, but not in the latter (Fig. 5D). Cleavage sites were present within the miR172 target sites of the majority (32/48) of sequences cloned from the Cly1.a RACE amplicon, and most of the cleavage sites were between the A and U nucleotides (Fig. 5E). However, most of the cly1.b amplicon clones were ribosomal RNAs or mRNAs of genes unrelated to Cly1, and only two of 48 clones contained sequence of Cly1 (Fig. 5E). Thus, it was conceivable that random mRNA breakage occurred in most of the cly1.b RACE products. Overall, it was shown that lodicule development and the resultant cleistogamy were brought about by a difference in cleavage of the Cly1 transcription product with miR172.

Note that since the miR172 target site was found in ESTs of not only barley, but also many other plants, it is conceivable that the cleavage of the transcription product with miR172 is present in wide varieties of plants (Fig. 6).

### (Example 6) Origin of cleistogamous barley

A phylogenetic analysis was conducted. ClustalW2 (http://www.ebi.ac.uk/Tools/clustalw2/) was used for amino acid alignment in this analysis. In addition, a phylogenetic tree was constructed by the neighbor joining method using PAUP4.0b10 (Swofford D PAUP*. Phylogenetic analysis using parsimony (*and other methods), ver.4. (Sinauer, Sunderland, Massachusetts, USA) 1998).

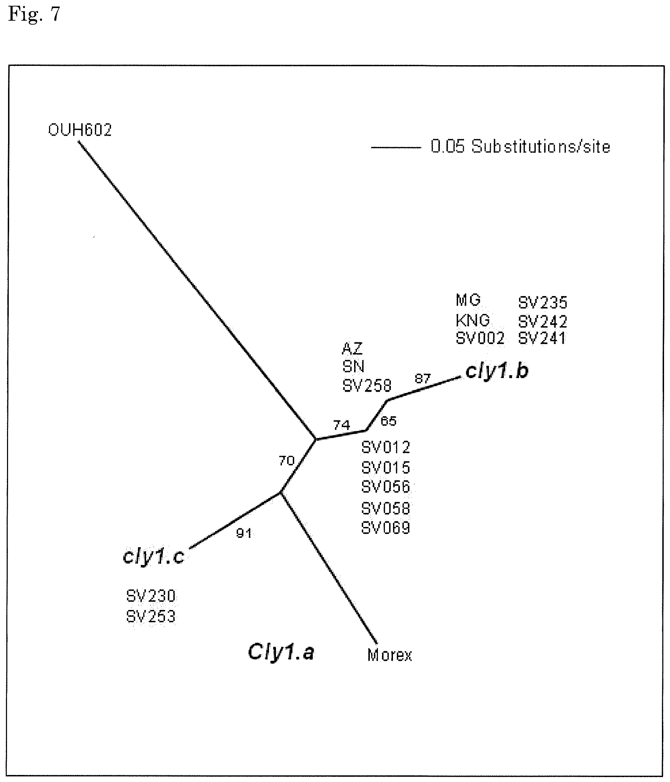

Two distinct lineages of cleistogamy were found by the phylogenetic analysis (Fig. 7). The KNG haplotype (cly1.b) differed from the wild-type AZ sequence by only two base substitutions, one of which was at position 3084 within the miR712 target sequence, and the other of which was at position 626 within the first exon (Fig. 8). The KNG haplotype appears to be directly originated from the wild-type AZ haplotype (Fig. 7). The AZ haplotype may be originated from the SV012 haplotype, because G at position 2252 is shared with noncleistogamous cultivars and wild-type barley (Fig. 8). The origin of the second cleistogamous haplotype (cly1.c) is uncertain. However, the two cleistogamous haplotypes appear to represent independent mutation events (Fig. 7). The 3-kb Cly1 coding sequence was invariant across both cleistogamous haplotypes, which suggests that the origin of cleistogamous barley was probably relatively recent.

### [Industrial Applicability]

As described above, according to the present invention, a novel gene Cly1/cly1 controlling chasmogamy/cleistogamy of a plant was identified, and it is made possible to determine chasmogamy/cleistogamy of a plant and to produce or breed a cleistogamous plant by making use of the identified Cly1/cly1 gene. The determination of chasmogamy/cleistogamy in the present invention uses the gene controlling the chasmogamy/cleistogamy as a target, and besides can be carried out by use of individuals at an early stage of cultivation (for example, seeds or seedlings). For this reason, the use of the determination method of the present invention makes it possible to specifically and efficiently breed a cleistogamous variety.

A cleistogamous plant produced or bred by use of the cly1 gene has enhanced resistance to Fusarium ear blight. Accordingly, the present invention can contribute to reduction of damage due to Fusarium ear blight. Moreover, pollen dispersal can also be prevented by imparting a cleistogamous trait to a plant. Recently, it has been pointed out that genes artificially modified by genetic recombination or the like may be dispersed to the natural world, so that the ecosystem of the natural world may be threaten, and the conservation of wild species may be affected. The introduction of cleistogamy to a plant is also effective for prevention of the dispersal.

### [Sequence Listing Free Text]

SEQ ID NO: 10
<223> microRNA
SEQ ID NO: 11
<223> microRNA target site
SEQ ID NOs: 12 to 16
<223> artificially synthesized primer sequences

## Claims

1. A DNA which does not undergo cleavage with a microRNA comprising the base sequence of SEQ ID NO: 10, and which imparts cleistogamy to a plant, wherein the DNA is described in any one of the following (a) to (c):
(a) a DNA comprising a coding region of the base sequence shown in SEQ ID NO: 1, 2, 4, or 5;
(b) a DNA comprising a base sequence in which one or a plurality of bases are substituted, deleted, added, and/or inserted in a coding region of the base sequence shown in SEQ ID NO: 1, 2, 4, 5, 7, or 8, and has a microRNA target site comprising a base sequence in which "a" at position 8 is substituted with "g" in comparison with the base sequence shown in SEQ ID NO: 11 or in which both "a"s at positions 2 and 14 are substituted in comparison with the base sequence shown in SEQ ID NO: 11; and
(c) a DNA which hybridizes with a DNA comprising the base sequence shown in SEQ ID NO: 1, 2, 4, 5, 7, or 8 under stringent conditions, and has a microRNA target site comprising a base sequence in which "a" at position 8 is substituted in comparison with the base sequence shown in SEQ ID NO: 11 or in which both "a"s at positions 2 and 14 are substituted with "g" in comparison with the base sequence shown in SEQ ID NO: 11.

2. The DNA according to claim 1, wherein the substitution of the bases in the microRNA target site does not involve change in any encoded amino acid.

3. A vector comprising the DNA according to claim 1 or 2.

4. A plant cell into which the DNA according to claim 1 or 2 is introduced as an exogenous DNA and which has the DNA in a homozygous manner by converting both cly1 alleles into the DNAs.

5. A plant comprising the cell according to claim 4.

6. A plant which is a progeny or a clone of the plant according to claim 5, and which has the DNA according to claim 1 or 2 in a homozygous manner and comprises none of chasmogamous-type Cly1 genes.

7. A propagation material of the plant according to claim 5 or 6, and which has the DNA according to claim 1 or 2 in a homozygous manner and comprises none of chasmogamous-type Cly1 genes.

8. A method for producing a plant having a cleistogamous trait, comprising a step of producing a plant which has the DNA according to claim 1 or 2 in a homozygous manner, by introducing the DNA as an exogenous DNA into the plant and converting both cly 1 alleles into the DNAs.

9. An agent for imparting a cleistogamous trait to a plant, comprising the DNA according to claim 1 or 2, or a vector into which the DNA is inserted.

10. A method for determining chasmogamy/cleistogamy of a plant, comprising:
comparing a base sequence of a DNA of a test plant with a reference base sequence, wherein the DNA is described in any one of the following (a) to (c):
(a) a DNA comprising a coding region of the base sequence shown in SEQ ID NO: 1, 2, 4, 5, 7, or 8;
(b) a DNA comprising a base sequence in which one or a plurality of bases are substituted, deleted, added, and/or inserted in a coding region of the base sequence shown in SEQ ID NO: 1, 2, 4, 5, 7, or 8, and has a microRNA target site comprising a base sequence in which "a" at position 8 is substituted in comparison with the base sequence shown in SEQ ID NO: 11 or in which both "a"s at positions 2 and 14 are substituted in comparison with the base sequence shown in SEQ ID NO: 11; and
(c) a DNA which hybridizes with a DNA comprising the base sequence shown in SEQ ID NO: 1, 2, 4, 5, 7, or 8 under stringent conditions, and has a microRNA target site comprising a base sequence in which "a" at position 8 is substituted in comparison with the base sequence shown in SEQ ID NO: 11 or in which both "a"s at positions 2 and 14 are substituted in comparison with the base sequence shown in SEQ ID NO: 11.

11. A method for determining chasmogamy/cleistogamy of a plant, comprising detecting cleavage, with a microRNA comprising the base sequence shown in SEQ ID NO: 10, of a transcription product of the DNA according to claim 1 or 2 of a test plant.

## Patentansprüche

1. Eine DNA, die mit einer microRNA, welche die Basensequenz mit der SEQ ID NO: 10 umfasst, keiner Spaltung unterliegt und welche einer Pflanze Kleistogamie verleiht, wobei die DNA von einer der folgenden a) bis c) beschrieben wird:
a) einer DNA, die eine Codierregion mit der in SEQ ID NO: 1, 2, 4 oder 5 gezeigten Basensequenz umfasst,
b) einer DNA, die eine Basensequenz umfasst, in welcher eine oder mehrere Basen in einer Codierregion mit der in der SEQ ID NO: 1, 2, 4, 5, 7 oder 8 gezeigten Basensequenz substituiert, deletiert, addiert und/oder insertiert sind, und welche einen microRNA-Zielort besitzt, der eine Basensequenz umfasst, bei welcher im Vergleich mit der in SEQ ID NO: 11 gezeigten Basensequenz "a" in Position 8 mit "g" substituiert ist, oder bei welcher im Vergleich mit der in SEQ ID NO: 11 gezeigten Basensequenz beide "a" in den Positionen 2 und 14 substituiert sind, und
c) einer DNA, die mit einer DNA, welche die in der SEQ ID NO: 1, 2, 4, 5, 7 oder 8 gezeigte Basensequenz umfasst, unter stringenten Bedingungen hybridisiert, und welche einen microRNA-Zielort besitzt, der eine Basensequenz umfasst, bei welcher im Vergleich mit der in SEQ ID NO: 11 gezeigten Basensequenz "a" in Position 8 substituiert ist, oder bei welcher im Vergleich mit der in SEQ ID NO: 11 gezeigten Basensequenz beide "a" in den Positionen 2 und 14 substituiert sind.

2. Die DNA nach Anspruch 1, wobei die Substitution der Basen an dem microRNA-Zielort keine Veränderung bei einer codierten Aminosäure bedeutet.

3. Ein Vektor, der die DNA nach Anspruch 1 oder 2 umfasst.

4. Eine Pflanzenzelle, in welche die DNA nach Anspruch 1 oder 2 als eine exogene DNA eingeschleust worden ist und welche die DNA auf eine homozygote Art und Weise besitzt, indem beide cly1-Allele in die DNAs umgewandelt worden sind.

5. Eine Pflanze, welche die Zelle nach Anspruch 4 umfasst.

6. Eine Pflanze, die ein Nachkomme oder Klon der Pflanze nach Anspruch 5 ist und welche die DNA nach Anspruch 1 oder 2 auf homozygote Art und Weise besitzt und keines der chasmogamen Clyl-Gene umfasst.

7. Ein Vermehrungsmaterial der Pflanze nach Anspruch 5 oder 6, welches die DNA nach Anspruch 1 oder 2 auf eine homozygote Art und Weise besitzt und keines der chasmogamen Cly1-Gene umfasst.

8. Ein Verfahren zur Herstellung einer Pflanze mit einem kleistogamen Merkmal, welches die Stufe der Erzeugung einer Pflanze, welche die DNA nach Anspruch 1 oder 2 auf homozygote Art und Weise besitzt, durch Einschleusen der DNA als eine exogene DNA in die Pflanze und Umwandeln beider clyl-Allele in die DNAs umfasst.

9. Ein Mittel, um einer Pflanze ein kleistogames Merkmal zu verleihen, welches die DNA nach Anspruch 1 oder 2 oder einen Vektor, in welchen die DNA insertiert ist, umfasst.

10. Ein Verfahren zum Bestimmen von Chasmogamie/Kleistogamie einer Pflanze, welches das Vergleichen einer Basensequenz einer DNA einer Versuchspflanze mit einer Referenzbasensequenz umfasst, wobei die DNA von einer der folgenden a) bis c) beschrieben wird:
a) einer DNA, die eine Codierregion mit der in SEQ ID NO: 1, 2, 4, 5, 7 oder 8 gezeigten Basensequenz umfasst,
b) einer DNA, die eine Basensequenz umfasst, in welcher eine oder mehrere Basen in einer Codierregion mit der in der SEQ ID NO: 1, 2, 4, 5, 7 oder 8 gezeigten Basensequenz substituiert, deletiert, addiert und/oder insertiert sind, und welche einen microRNA-Zielort besitzt, der eine Basensequenz umfasst, bei welcher im Vergleich mit der in SEQ ID NO: 11 gezeigten Basensequenz "a" in Position 8 substituiert ist, oder bei welcher im Vergleich mit der in SEQ ID NO: 11 gezeigten Basensequenz beide "a" in den Positionen 2 und 14 substituiert sind, und
c) einer DNA, die mit einer DNA, welche die in der SEQ ID NO: 1, 2, 4, 5, 7 oder 8 gezeigte Basensequenz umfasst, unter stringenten Bedingungen hybridisiert, und welche einen microRNA-Zielort besitzt, der eine Basensequenz umfasst, bei welcher im Vergleich mit der in SEQ ID NO: 11 gezeigten Basensequenz "a" in Position 8 substituiert ist, oder bei welcher im Vergleich mit der in SEQ ID NO: 11 gezeigten Basensequenz beide "a" in den Positionen 2 und 14 substituiert sind.

11. Ein Verfahren zum Bestimmen der Chasmogamie/Kleistogamie einer Pflanze, welches die Detektion einer Spaltung eines Transkriptionsprodukts der DNA nach Anspruch 1 oder 2 einer Versuchspflanze durch eine microRNA, welche die in SEQ ID NO: 10 gezeigte Basensequenz umfasst, umfasst.

## Revendications

1. ADN qui ne subit pas de clivage avec un microARN, comprenant la séquence de bases de SEQ ID N°10, et qui confère la cléistogamie à une plante, où l'ADN est décrit par l'un quelconque des points (a) à (c) suivants :
(a) un ADN comprenant une région codante de la séquence de bases représentée en SEQ ID N°1, 2, 4 ou 5 ;
(b) un ADN comprenant une séquence de bases, dans laquelle une ou une série de bases est substituée, délétée, ajoutée et/ou insérée dans une région codante de la séquence de bases représentée en SEQ ID N°1, 2, 4, 5, 7 ou 8, et qui a un site cible de microARN, comprenant une séquence de bases, dans laquelle « a » en position 8 est substitué par « g » par comparaison à la séquence de bases donnée en SEQ ID N°11 ou dans laquelle les deux « a » en positions 2 et 14 sont substitués par comparaison à la séquence de bases donnée en SEQ ID N°11, et
(c) un ADN qui s'hybride à un ADN comprenant la séquence de bases représentée en SEQ ID N°1, 2, 4, 5, 7 ou 8 dans des conditions rigoureuses, et qui a un site cible de microARN, comprenant une séquence de bases, dans laquelle « a » en position 8 est substitué par comparaison à la séquence de bases donnée en SEQ ID N°11 ou dans laquelle les deux « a » en positions 2 et 14 sont substitués par « g » par comparaison à la séquence de bases donnée en SEQ ID N°11.

2. ADN selon la revendication 1, où la substitution des bases dans le site cible de microARN n'implique pas un changement au niveau des acides aminés codés.

3. Vecteur comprenant l'ADN selon la revendication 1 ou 2.

4. Cellule végétale dans laquelle l'ADN selon la revendication 1 ou 2 est introduit comme ADN exogène et qui a l'ADN de manière homozygote par conversion des deux allèles cly1 dans les ADN.

5. Plante comprenant la cellule selon la revendication 4.

6. Plante qui est la descendance ou un clone de la plante selon la revendication 5, qui porte l'ADN selon la revendication 1 ou 2 de manière homozygote et qui ne comprend aucun des gènes Cly1 de type chasmogamie.

7. Matériau de propagation de la plante selon la revendication 5 ou 6, qui porte l'ADN selon la revendication 1 ou 2 de manière homozygote et qui ne comprend aucun des gènes Cly1 de type chasmogamie.

8. Procédé de production d'une plante ayant un trait cléistogame, comprenant une étape de production d'une plante qui porte l'ADN selon la revendication 1 ou 2 de manière homozygote par introduction de l'ADN comme ADN exogène dans la plante et conversion des deux allèles cly1 dans les ADN.

9. Agent pour conférer un trait cléistogame à une plante, comprenant l'ADN selon la revendication 1 ou 2, ou un vecteur dans lequel l'ADN est inséré.

10. Procédé de détermination de la chasmogamie/ cléistogamie d'une plante, comprenant :
la comparaison d'une séquence de bases d'un ADN d'une plante test avec une séquence de bases de référence, où l'ADN est décrit par l'un quelconque des points (a) à (c) suivants :
(a) un ADN comprenant une région codante de la séquence de bases représentée en SEQ ID N°1, 2, 4, 5, 7 ou 8 ;
(b) un ADN comprenant une séquence de bases, dans laquelle une ou une série de bases est substituée, délétée, ajoutée et/ou insérée dans une région codante de la séquence de bases représentée en SEQ ID N°1, 2, 4, 5, 7 ou 8, et qui a un site cible de microARN, comprenant une séquence de bases, dans laquelle « a » en position 8 est substitué par comparaison à la séquence de bases donnée en SEQ ID N°11 ou dans laquelle les deux « a » en positions 2 et 14 sont substitués par comparaison à la séquence de bases donnée en SEQ ID N°11, et
(c) un ADN qui s'hybride à un ADN comprenant la séquence de bases représentée en SEQ ID N°1, 2, 4, 5, 7 ou 8 dans des conditions rigoureuses, et qui a un site cible de microARN, comprenant une séquence de bases, dans laquelle « a » en position 8 est substitué par comparaison à la séquence de base donnée en SEQ ID N°11 ou dans laquelle les deux « a » en positions 2 et 14 sont substitués par comparaison à la séquence de base donnée en SEQ ID N°11.

11. Procédé de détermination de la chasmogamie/ cléistogamie d'une plante, comprenant la détection d'un clivage, avec un microARN comprenant la séquence de bases représentée en SEQ ID N°10, du produit de transcription de l'ADN selon la revendication 1 ou 2 dans une plante test.
